# EUROPEAN PATENT APPLICATION

(11) **EP 4 342 454 A1**
(43) Date of publication of application: **27.03.2024**
(21) Application number: 23198035.0
(22) Date of filing: 18.09.2023
(51) Int. Cl.: A61K 9/00, A61K 9/10, A61K 31/713, A61K 47/02, A61K 47/12, A61K 47/26, A61K 47/34

(54) **NOVEL COMPOSITIONS BASED ON POLYINOSINIC-POLYCYTIDYLIC ACID**

(30) Priority: 20.09.2022 US 202263408144 P; 11.07.2023 US 202318349989
(71) Applicant: Highlight Therapeutics, S.L., 46980 Valencia (ES)
(72) Inventor: FUNES, Juan-Manuel, 46980 Paterna, Valencia (ES); POZUELO RUBIO, Mercedes, 46980 Paterna, Valencia (ES); QUINTERO ORTIZ, Marisol, 46980 Paterna, Valencia (ES)
(74) Representative: Grund, Martin

(57) **Abstract**

An aqueous formulation, comprising particles formed by a combination of polyinosinic-polycytidylic acid [Poly(I:C)] molecules and polyethyleneimine, wherein the formulation comprises a pH of between pH 5.0 and pH 7.5. Said composition for use in therapy, wherein the therapy is stimulating an immune response, treating or preventing cancer, or a vaccine therapy.

## Description

### FIELD OF THE INVENTION

The present invention relates to novel pharmaceutical formulations and compositions and related methods for administering the same, in particular microparticles comprising polyinosinic-polycytidylic acid.

### BACKGROUND OF THE INVENTION

Polyinosinic-polycytidylic acid [Poly(I:C)] is a molecule that is formed by the pairing of synthetic, single-stranded nucleic acids, namely polyinosinic acid and polycytidylic acid, thereby producing populations of double-stranded nucleic acids featuring different lengths and structures. Poly(I:C) molecules can be optimally formulated as particles comprising synthetic polymers for effectively achieving a desired therapeutic or prophylactic effect. Such effects may include the stimulation of the release or production of cytokines and the activation of TLR3 (Toll-like receptor 3) and/or of other biological pathways triggering sequence-independent, immunostimulatory activities that can further be exploited to prepare anti-infective, anticancer, and/or adjuvant agents that can be administered alone or in combination with other chemical or biological compounds, including nucleic acid-based cancer vaccines (Bishani A and Chernolovskaya E, 2021).

These therapeutic or prophylactic effects of clinical interest are at least partly dependent on the methods employed for administering such formulations and related pharmaceutical compositions containing particles that are formed by Poly(I:C) molecules and synthetic polymers. The pharmaceutical compositions and formulations may contain particles that are produced and validated according to appropriate controlled size, stability, and bioavailability characteristics. However, such particles may present an undesirable tendency to form aggregates during storage or other manipulation. If not eliminated from the composition (for instance, by filtration), these larger aggregates might adversely impact the therapeutic properties of the particles and their use in a variety of clinical applications.

Several technologies and compounds have been used to improve the stability, bioavailability, or dosage of pharmaceutical compositions presenting chemical properties that make them particularly unsuitable for intravenous or subcutaneous injection, or other specific routes of administration involving injection, infusion, or implantation (Badkar A et al., 2021; Gutierrez L et al., 2020). Such approaches are frequently employed to avoid pain or other discomfort during administration, in particular at the injection site when the injected volume of the formulations is larger than 1.0 ml (Usach I et al. 2019).

BO-112 is one example of a Poly(I:C) formulation that is formed by Poly(I:C) molecules complexed with polyethyleneimine to form nanoplexed particles. This formulation has been successfully tested in several animal models and clinical protocols where it is typically administered intratumorally, in particular in combination with checkpoint inhibitors (Agrawal E and Kandimalla R, 2019; Huang L et al., 2022; Aznar M et al., 2019; Márquez-Rodas I et al., 2022). The literature disclosing the preparation, administration, and medical use of BO-112 and similar Poly(I:C)-based formulations, which are generally referred to as BO-11X compositions (WO 2017/085228; WO 2018/210439; WO 2020/104628). However, these publications do not disclose a means for providing these formulations in a form and pH that is suitable for most types of parenteral administration, especially in cases where certain clinical protocols and dosages require administering the BO-11X formulations at a pH closer to a standard physiological range.

Accordingly, there is a need for methods of effectively formulating and administering BO-11X compositions in a manner that maintains the biophysical and biological efficacy of these compositions as initially validated, thereby minimizing adverse reactions at the administration site, especially when these compositions are administered in large volumes, e.g., as a plurality of injections or infusions and/or that can be administered at a low flow rate over a long period of time.

### SUMMARY OF THE INVENTION

The present invention relates to aqueous formulations that are suitable for the administration of Poly(I:C) molecules, in particular in the form of microparticles comprising Poly(I:C) molecules, e.g., as an infusible or injectable formulation for subcutaneous, intravenous, intratumoral, intralesional, or intramuscular administration, in a manner that avoids inducing adverse local reactions at the site of administration, in particular when administered in volumes larger than 1.0 ml, administered as several injections, and/or that can be administered using a low injection flow rate. In one embodiment, these formulations are generated by mixing a composition comprising particles that are formed by polymers and Poly(I:C) molecules using a deacidifying solution that is selected among established clinically acceptable buffers useful for drug administration. The selection of the type, concentration, and volume of these buffers is made not only to promote the compatibility of the final pharmaceutical composition according to a variety of clinical uses, but also to ensure a limited impact on the specific biophysical and biomedical characteristics of the BO-11X formulations as initially prepared and validated, including the features of particle size, the amount, and/or the variable length of the Poly(I:C) molecules that are administered, either shortly following formulation preparation and/or after long-term storage at an appropriate temperature.

In one embodiment, the aqueous formulations according to the invention comprise polymer-based microparticles, in particular those formed by a combination of polyethyleneimine molecules and Poly(I:C) molecules. The characteristics of the polyethyleneimine molecules and of the Poly(I:C) molecules, as well as of the microparticles and the methods for producing same, are defined in the literature as reference formulations comprising polymer-based microparticles referred to as, for instance, BO-11X compositions. One example of such a composition is referred to as a BO-112 composition (i.e., as disclosed in WO 2017/085228, WO 2018/210439, and WO 2020/104628, which are incorporated herein by reference in their entirety), wherein Poly(I:C) molecules are defined according to particular size ranges (e.g., at least 40% of the Poly(I:C) molecules have at least 850 base pairs, and at least 50% of Poly(I:C) molecules have between 400 and 5000 base pairs). When modified according to the present invention, the resulting BO-11X and BO-112 compositions may be provided as novel formulations, referred to herein as BO-11XSQ and BO-112SQ formulations, respectively, which are optimized for injection, for example, parenteral administration through the skin or directly into the bloodstream, into a particular tissue, or into a tumor.

In some embodiments according to the invention, the BO-11XSQ formulations can be prepared by mixing a BO-11X composition with a deacidifying solution that is selected from a range of buffers already established for a given clinical use, for instance, wherein the mixing occurs prior to administration of the composition. In some preferred embodiments, the deacidifying solution has a pH of 6 or greater, e.g., pH 6, 7, 8, 9, 10, 11, 12, 13 and 14, including all fractional values therebetween, preferably between pH 6 and pH 12 and all values therebetween, even more preferably between pH 7.0 and pH 10.0 and all values therebetween, which can effectively increase the initial pH of the BO-11X composition from a range of between pH 2.0 and pH 4.0 to a pH of 4.5 or greater. In some embodiments, the pH of a BO-11XSQ formulation is preferably between pH 4.5 and pH 8.5 including all values therebetween, more preferably between pH 5.0 and pH 7.5, and even more preferably between pH 5.0 and pH 6.5. When compared to the characteristics of the initial BO-11X composition, the desired pH value of a BO-11XSQ formulation is associated with a minimal loss of Poly(I:C) molecules, for instance, less than a 10%, 5%, 2%, or 1% loss with minimal effects on particle aggregation and cancer-specific cytotoxicity. These characteristics can be measured and compared by using various assays and equipment that are commonly available for measuring the relevant values to compare the effects of clinically acceptable buffers acting as deacidifying solutions for the BO-11X compositions. One exemplary deacidifying solution is sodium bicarbonate, which can be used at a high concentration for producing the disclosed BO-11XSQ formulations with minimal impact on the volume and other features of the initial BO-11X composition.

In one preferred embodiment, the volume of the deacidifying solution that is added to a BO-11X composition for producing a BO-11XSQ formulation as disclosed herein is less than 10.0%, 8.0%, 5.0%, 3.0%, 2.0%, 1.0%, 0.5%, 0.2%, 0.1%, 0.05%, 0.02%, or 0.01% of the volume of the BO-11X composition. In some embodiments, the volume of the BO-11X composition may be between 0.1 ml and 25 ml, or greater, for instance, up to 50 ml including all values therebetween. In other embodiments, the Poly (I:C) molecules may be present in the composition at a concentration of at least 0.1, 0.2, 0.5, 0.7, or 1.0 mg/ml. The volume of deacidifying solution is consequently present at a volume of between 0.00001 ml and 5 ml. Alternatively, in other embodiments, the amount of deacidifying solution to be added can be defined according to the final concentration in a BO-11XSQ formulation disclosed herein, for instance, defined according to a certain percentage, for instance, less than about 10%, 5.0%, 1.0%, or 0.1% or according to a certain molar concentration, for instance, less than 0.2M, 0.1M, 0.05M, or 0.01M. In some embodiments according to the present invention, the BO-11XSQ formulations may be used within 1 minute, 2 minutes, 5 minutes, 10 minutes, 15 minutes, 30 minutes, 60 minutes, 120 minutes or more following preparation thereof, preferably using a deacidifying solution and a BO-11X composition, whereby the BO-11XSQ formulations may be filtered (or not) prior to administration.

In another embodiment, the BO-11XSQ formulations are distributed for clinical use as a batch preparation or as one or more aliquots ready for single use. In some embodiments, the batch preparation or one or more aliquots are comprised within a kit or kit of parts. In other embodiments, the deacidifying solution and a BO-11X composition may be provided in a kit or kit of parts comprising preparations of both components, either as batch preparations or as aliquots for preparing a single dose to be administered, for example, by injection or infusion. In yet other embodiments, the kit may contain either component already aliquoted for injection while the other component is provided as a batch preparation from which the desired amount of solution is added to the aliquot prior to administration. In a preferred embodiment, the kit comprises a BO-11X composition already aliquoted for injection and a deacidifying solution provided as a batch preparation. In some embodiments, the kit may further comprise one or more further items such as syringes, needles, filters, vials, diluents, a local anesthetic solution, and/or instructions for preparing and/or using a BO-11XSQ formulation, and in particular BO-112SQ formulations.

In preferred embodiments, the BO-11XSQ formulations disclosed herein are administered by injection, for example, in accordance with established clinical protocols and/or routes of administration. The BO-11XSQ formulations according to the instant invention can be used as a medicament, preferably as a pharmaceutical composition for e.g., treating cancer or as an adjuvant in a variety of vaccination protocols against cancer cell or infectious agents. The BO-11XSQ formulations herein can be administered using regimens, dosages, and in combination with a vaccine or a therapeutic agent, for instance, as described for BO-11X compositions as disclosed in WO 2017/085228, WO 2018/210439, and WO 2020/104628. Specific novel dosages and regimens can be established using a disclosed BO-11XSQ formulation that is administered according to a particular route.

The present BO-11XSQ formulations are preferably administered intravenously, intratumorally, intramuscularly, or subcutaneously, using, e.g., a syringe, a pump, or other appropriate device. The volume of the BO-11XSQ formulation that is to be administered is preferably the same volume or a fraction thereof, which has been prepared using a deacidifying solution and a BO-11X composition as described herein, which may be comprised between a 0.1 ml and 10 ml volume or more, preferably between 1 ml and 5 ml. In some embodiments, the mode of administration is by subcutaneous, intratumoral, intramuscular, or intravenous injection or infusion, for use in a variety of clinical applications, for instance, for treating cancer. The BO-11XSQ formulations herein may be administered into the skin, mucosae, tongue, connective tissues, muscles, joints, thigh region, upper arm, abdominal region, gluteal region, or scapular region of the subject, after or before administration of a local anesthetic solution, as described in the literature for such methods of administration.

In some embodiments, the disclosed BO-11XSQ formulations can be administered, for instance, as an adjuvant or for providing additional clinical effects, e.g., overcoming resistance to another drug, in combination with a second pharmaceutical composition, for example, a pharmaceutical composition comprising an anti-cancer drug or a vaccine. The BO-11XSQ formulations and the second pharmaceutical composition may be administered as separate formulations, simultaneously or sequentially (in any order), and using any appropriate route of administration, e.g., orally, subcutaneously, topically, intra- or transdermally, intratumorally, intravenously, intramuscularly, intrathecally, intranasally, intra-articularly, or intravesically. In other embodiments, the disclosed BO-11XSQ formulations and the second pharmaceutical composition may be mixed and administered as a single composition by injection or infusion.

In cases where the disclosed BO-11XSQ formulations are being administered in applications for treating or preventing cancer, the regimen and the treatment protocols may be performed similarly to what is described for known BO-11X formulations, and may be administered in combination with a treatment or drug for treating or preventing cancer, for example, a solid cancer, a blood cancer, or cancer metastases. The BO-11XSQ formulations herein may be administered before or after administering a second pharmaceutical composition. In some embodiments, the second pharmaceutical composition is preferably a vaccine, for instance, a cell-, peptide-, or nucleic acid-based vaccine useful for delivering cancer antigens and generating a cancer-specific immunological response in the subject, an anti-cancer treatment, or a standard-of-care medical treatment. In some embodiments, the second pharmaceutical composition comprises an anti-cancer agent or a standard anti-cancer treatment such as surgery, radiation therapy, tumor ablation, or cryotherapy. In addition, the anti-cancer agent may target or inhibit specific proteins expressed by cancer cells (e.g., metabolic enzymes or proteins that are involved in DNA repair and/or replication) or by cells in the immune system such that the cancer is eliminated by the immune system, i.e., immunotherapy. In the latter case, the therapy may comprise the administration of a second pharmaceutical composition comprising one or more immune-modulating agents including a monoclonal antibody that targets PD-1, PD-L1 , PD-L2, CTLA-4, CD137, OX-40, TIM3, or LAG3, a cell-based product (e.g., CAR-T cells), a cell-, peptide-, or nucleic acid-based cancer vaccine, or any agent that can target an immune cell, e.g., dendritic cells or regulatory T cells.

The regimen and dosage of the BO-11XSQ formulations described herein, in addition to their use in combinations with other therapeutic agents, in particular anti-cancer drugs, are based on those reported for known BO-11X compositions, for example, as disclosed in WO 2017/085228, WO 2018/210439, and WO 2020/104628, for treating, reducing, ameliorating, or preventing cancer growth, survival, metastasis, epithelial-mesenchymal transition, drug resistance or insensitivity, immunologic escape or recurrence.

Notably, the use of the BO-11XSQ formulations as disclosed herein may be specifically improved in order to enhance the efficacy and/or improve the clinical acceptability of the agent or therapy that is being combined with the BO-11XSQ formulation, in particular after determining that a subject is resistant, insensitive, or poorly or not responding to the second pharmaceutical composition. The BO-11XSQ administration may also result in a statistically and therapeutically significant increase in the number of circulating immune cells (e.g., CD8+ memory T cells or CD4+T regulatory cells, myeloid cells, dendritic cells, and the like) or in the concentration of cytokines (e.g., Interleukin-6, Interferon beta, Interferon gamma, or TNF alpha) in the peripheral blood, serum, or plasma of the subject.

The activities of clinical interest and pharmacokinetics of the particles that are administered using a BO-11XSQ formulation (or any BO-11X formulation) may be also determined by methods that allow evaluating the presence of Poly(I:C) molecules in biological samples, in particular using peripheral blood, serum, or plasma of the subject that has been treated with the instant formulations. These methods generally involve a controlled, *in vitro* disaggregation of the particles within the biological sample using heat or certain compounds that can influence the electrostatic interactions between the Poly(I:C) molecules and the synthetic polymers (e.g., polysaccharide or glycosaminoglycans, in particular heparin), and/or compounds that influence hydrophobic interactions by disrupting hydrogen bonding within macromolecules (e.g., chaotropic agents, in particular guanidinium chloride and/or urea). The denaturation, dissociation, or disaggregation step is followed by the isolation and subsequent identification of the Poly(I:C) molecules using antibodies directed against certain components characterizing these molecules (e.g., double-stranded ribonucleotides, dsRNA). For instance, the detection and quantification of complexes formed by Poly(I:C) molecules and antibodies may be performed using kits or assays, e.g., enzyme-linked immunosorbent assay (ELISA) or other antibody-based protocols based on a solid or liquid phase that permits quantifying the antibody-bound molecules by fluorometric, luminescent, or colorimetric methods.

Further embodiments, such as formulations, uses and methods according to the present invention, are disclosed in additional detail and exemplified in the Examples herein.

### DETAILED DESCRIPTION OF THE INVENTION

### Definitions and Preparation of the BO-11XSQ Formulations

The presently disclosed BO-11XSQ formulations are prepared by adding a deacidifying solution to previously described BO-11X compositions, the latter being Poly(I:C)-based compositions that show agonist effects on proteins acting as a double-stranded RNA intracellular sensor, including TLR3 (Toll-like Receptor 3) and/or MDA5 (WO 2017/085228; WO 2018/210439). The BO-11XSQ formulations described herein may further comprise particles formed by specific combinations of Poly(I:C) molecules and polyethyleneimine molecules. The particles of the instant BO-11XSQ formulations may further comprise characteristics that are common to those of known BO-11X compositions, in particular with respect to the respective components and the size distribution of, and content in, the Poly(I:C) molecules, which make the compositions particularly suitable for clinical applications and also compatible with GMP (Good Manufacturing Practices) requirements.

In some embodiments, the particles of the BO-11XSQ formulations herein may contain polyethyleneimines that are at least 95% linear polyethyleneimines and preferably have an average molecular weight between 17 and 23 kDa, with a polydispersity index lower than 1.5, and preferably below 1.0. The Poly(I:C) molecules (or a salt or solvate thereof) may present a range of sizes that are disclosed in WO 2017/085228 or WO 2018/210439, that is, at least 40% of the Poly(I:C) molecules having at least 850 base pairs, and at least 50%, 60%, 70%, 75%, or 80% of Poly(I:C) molecules having between 400 and 5000 base pairs. Alternatively, at least 50% of the Poly(I:C) molecules in the formulation can have at least between 400 and 5000 base pairs, and at least 50%, 60%, 70%, or 75% of Poly(I:C) molecules can have more than 850 base pairs. The sizes for Poly(I:C) molecules in the BO-11XSQ formulations may be defined using other ranges, in particular lower than 400 base pairs, between 400 and 850 base pairs, between 850 and 5000 base pairs, and more than 5000 base pairs. In preferred embodiments, the following ranges can apply to the BO-11XSQ formulations disclosed herein:
a) between 5% and 60%, preferably between 10% and 30% and more preferably between 12% and 25% of Poly(I:C) molecules have less than 400 base pairs;
b) between 15% and 45%, preferably between 15% and 30% (or between 20% and 45%), and more preferably between 20% and 30% of Poly(I:C) molecules have between 400 and 850 base pairs;
c) between 20% and 70%, preferably between 50% and 70%, and more preferably between 55% and 65% of Poly(I:C) molecules have between 850 and 5000 base pairs; and
d) between 0% and 10%, preferably between 1% and 7%, and more preferably between 2% and 5% of Poly(I:C) molecules have more than 5000 base pairs.

The foregoing sizes and percentages may be established using known methods and equipment such as those based on Gel Permeation Chromatography (GPC), Agarose gel Electrophoresis (AGE), or other approaches affording a similar level of accuracy.

These technologies may also be used for determining the ranges of sizes for the single-stranded polyinosinic acid (Poly(I) molecules) and polycytidylic acid (Poly(C) molecules, which are separately manufactured and then used for preparing the BO-11X compositions as described in WO 2017/085228 and WO 2018/210439. In preferred embodiments, the following ranges can apply to the Poly(I) molecules:
a) between 70% and 95%, preferably between 70% and 90% and more preferably between 70% and 85% of Poly(I) molecules have less than 400 bases;
b) between 5% and 25%, preferably between 5% and 20% and more preferably between 10% and 20% of Poly(I) molecules have between 400 and 850 bases;
c) between 0% and 10%, preferably between 1% and 7%, and more preferably between 2% and 5% of Poly(I) molecules have between 850 and 5000 bases; and
d) between 0% and 5%, preferably between 0% and 3%, and more preferably between 0% and 1% of Poly(I) molecules have more than 5000 bases.

Similarly, in preferred embodiments, the following ranges can apply to the Poly(C) molecules:
a) between 20% and 80%, preferably between 25% and 70% and more preferably between 30% and 60% of Poly(C) molecules have less than 400 bases;
b) between 15% and 40%, preferably between 25% and 40% and more preferably between 30% and 40% of Poly(C) molecules have between 400 and 850 bases;
c) between 3% and 50%, preferably between 5% and 40%, and more preferably between 10% and 25% of Poly(C) molecules have between 850 and 5000 bases; and
d) between 0% and 5%, preferably between 0% and 3%, and more preferably between 1% and 3% of Poly(I) molecules have more than 5000 bases.

The particles in the BO-11XSQ formulations according to the present invention preferably have a mono-modal diameter distribution. In particular, at least 95% or 99% of the particles have a diameter of less than or equal to 600 nm, and preferably less than or equal to 400 nm and more preferably below 300 nm (e.g., with at least of the particles having a diameter below 250 nm) and such particles have a mono-modal diameter distribution below this size limitation. The particles have a z-average diameter of less than or equal to 250 nm, 200 nm, or 150 nm, preferably between 30 nm and 150 nm, and more preferably between 60 nm and 130 nm, as measured according to ISO 22412:2017, with a median diameter (D50%) of between 75 nm and 150 nm, and preferably at least a D50% of 85 +/- 20 nm, and a polydispersity index of the particle diameter that is less than 1.5, and preferably inferior to 1.0, 0.6, 0.3, or 0.1.

The presently disclosed BO-11XSQ formulations may present additional characteristics, for example, a concentration of at least 0.1, 0.3, 0.5, 0.7, or 1.0 mg of Poly(I:C) molecules per ml of the total volume of the composition, and the presence of glucose or mannitol as an excipient, for instance, at a concentration of between 1 and 10% weight/volume of the BO-11XSQ formulation, preferably between 3% and 7%, more preferably 5%. The BO-11XSQ formulations herein, in particular those presenting an amount of Poly(I:C) molecules per ml and glucose or mannitol as an excipient, can be prepared according to the protocols described in WO 2017/085228 and WO 2018/210439 to provide the initial BO-11X composition. Accordingly, the particles in the BO-11XSQ formulations can be formed at a ratio of the number of moles of nitrogen of linear-polyethyleneimine to the number of moles of phosphorus of Poly(I:C) molecules that is equal to or greater than 2.5, preferably between 2.5 and 4.5, and more preferably between 2.5 and 3.5. During and/or after the process of manufacturing the composition, the intermediate or final solutions may be filtered through a filter having a pore diameter of less than or equal to 800 nm, 700 nm, 500 nm, 400 nm, or 300 nm. The BO-11XSQ formulations can present an osmolality of between 200 and 600 mOsm/kg, preferably between 220 and 340 mOsm/kg, and more preferably between 300 and 310 mOsm/kg. Moreover, the zeta potential of a BO-11XSQ formulation may be equal to or above 30mV, preferably between 35 and 50 mV, more preferably between 40 and 50 mV, and preferably around 40mV, i.e., as measured according to ISO 13099-2:2012.

The BO-11XSQ formulations herein, and in particular the specific formulation identified as a BO-112SQ formulation, differ from the corresponding BO-11X and BO-112 formulations by their pH value. While BO-11X and BO-112 formulations have a pH comprised of between 2.0 and 4.0, preferably between 2.7 and 3.4, such as a pH of 3.0 +/-0.2, the presently disclosed BO-11XSQ formulations present a higher pH value of equal to or greater than 4.5, preferably between 5.0 and 7.5, more preferably between 5.0 and 6.5. Notably, when compared to the original BO-11X and BO-112 compositions, the present BO-11XSQ formulations advantageously show a minimal loss of Poly(I:C) molecules, for instance, a loss of less than 15%, 10%, 5%, 2%, or 1%, with minimal effects on particle aggregation and cancer-specific cytotoxicity.

The Examples herein describe the assays and acceptable ranges of values for validating a deacidifying solution contemplated as an appropriate buffer to be added to a BO-11X composition for preparing the instant BO-11XSQ formulations. The selection of the deacidifying solution can be made amongst any buffer that is commonly used for pharmaceutical preparations destined for parenteral administration and further present a pH buffering range comprising at least between pH 5 and pH 6, between pH 5 and pH 7, or between pH 5 and pH 8. Such buffers should be optimally available at a sufficiently high concentration so that they can exert their activity when added in very low amounts, with minimal effect on the total volume of the BO-11XSQ and BO-112SQ formulations, thus without a meaningful dilution of the content of polymer-based microparticles and Poly(I:C) molecules in the original BO-11X and BO-112 formulations.

Among such buffers, deacidifying solutions containing citrate, succinate, phosphate, histidine, bicarbonate, and/or acetate are preferred. Buffers based on citrate, phosphate, and/or bicarbonate are most preferred, in particular when provided as a salt with sodium, magnesium, ammonium, or potassium, such as sodium bicarbonate. Moreover, the deacidifying solution is preferably a commercially available buffer that is registered for clinical use, and in particular a buffer that is listed in one of the pharmacopeia that are published under the authority of a government or a medical or pharmaceutical society, such as the United States Pharmacopeia (USP), the European Pharmacopoeia (Pharmacopoeia Europaea, Ph. Eur.), the Japanese Pharmacopoeia, the International Pharmacopoeia (Pharmacopoeia Internationalis, Ph. Int.), or other equivalent national Pharmacopeia or list of officially approved medical compositions.

A deacidifying solution that can act as a buffer or pH modifier according to the present invention may be added to a BO-11X composition for producing a BO-11XSQ formulation herein in an amount that can be determined using different criteria. As a first criterion, the volume of the deacidifying solution is preferably less than 10.0%, 5.0%, 3.0%, 2.0%, or 1.0% of the volume of the original BO-11X composition (e.g., between 5.0% and 0.01%, preferably between 3.0% and 0.01%). However, when the deacidifying solution is provided at a high concentration (e.g., at 1M or more), the volume of the deacidifying solution may be lower than 1.0%, such as 0.8%, 0.5%, 0.2%, 0.1%, 0.05%, 0.02%, or 0.01% of the volume of the initial BO-11X composition.

The original BO-11X composition can be provided in a volume that is preferably comprised of between 0.1 ml and 25 ml, for instance, 0.5, 1, 2, 3, 5, 10, 12, 15, or 20 ml, but more preferably in a volume suitable for injection, e.g., a volume comprised of between 1.0 and 12 ml, including 1.0, 2.0, 3.0, 5.0, or 8.0 ml, with a concentration of Poly (I:C) molecules of at least 0.1, 0.3, 0.5, 0.7, or 1.0 mg/ml. The volume of deacidifying solution to be added to the original BO-11X composition for producing the BO-11XSQ formulations herein can be consequently comprised of between 0.0001 ml and 5 ml, preferably between 0.01 ml and 2.5 ml. Even more preferably, in particular when the deacidifying solution is added to a volume of the original BO-11X composition that is suitable for a single injection or that is a volume to be distributed to clinical site (e.g., between 0.5 ml and 12 ml), the volume of the deacidifying solution to be added to the original BO-11X composition for producing the BO-11XSQ formulations herein is comprised of between 0.01 ml and 1 ml, or preferably between 0.05 ml and 0.5 ml. The resulting BO-11XSQ formulations then contain an amount of Poly (I:C) molecules of between 0.1 mg and 25 mg or more, but preferably between 0.1 mg and 15 mg, and more preferably between 0.5 mg and 8 mg, for instance, 0.6 mg, 1.0 mg, 1.5 mg, 2.0 mg, 3.0 mg, 5.0 mg, 7.0 mg, 10 mg, or 12 mg, which can be administered for each single administration of a BO-11XSQ formulation.

The amount of deacidifying solution to be added to a BO-11X composition for producing a BO-11XSQ formulation according to the present invention may be also defined by its final concentration in a BO-11XSQ formulation according to percentage (w/w), for instance, less than 2.0%, preferably less then 1.0%, (e.g., less than 0.5% or 0.2%) but more preferably less than 0.1% (e.g., less than 0.05%, 0.02%, or 0.01%). Alternatively, the amount of deacidifying solution to be added to a BO-11X composition for producing a BO-11XSQ formulation herein can be defined by its final molar concentration in the BO-11XSQ formulation, e.g., less than 0.2M, preferably less than 0.1M (e.g., less than 0.05M or 0.02M, e.g., between 0.2M and 0.01M), but more preferably less than 0.01M (e.g., between 0.009M and 0.0001M or between 0.005M and 0.001M).

The preparation of the BO-11XSQ formulations herein using a BO-11X composition is preferably performed by adding an appropriate volume of the deacidifying solution into a vial containing the BO-11X composition, followed by agitating, vortexing, inverting and the like that allows for mixing of the two solutions to achieve a uniform pH and particle distribution prior to use or storage of the mixture. The two solutions may be also added in the opposite order, i.e., an aliquot of BO-11X composition may be added to a vial containing the deacidifying solution, or by using a syringe in which either solution is already present and then using the needle to draw the other solution into the syringe and mixing the syringe prior to use or storage. The resulting BO-11XSQ formulation may be further manipulated prior to administration. For example, the content of the vial or syringe may be filtered to eliminate any particle aggregate or contaminant that may be present in the formulation, using, for instance, filters approved for pharmaceutical use with the appropriate pore size, e.g., 600 nm, 500 nm, 450 nm, 300 nm, 220 nm, or 200 nm.

The BO-11XSQ formulations according to the present invention may be modified using additional components of pharmaceutical interest that may improve the stability of the formulations for long-term storage (for instance, storage at a temperature of 4°C, 0°C, or at a temperature below 0°C), for administration, or *in vivo* efficacy of the BO-11XSQ formulation, and/or that may provide additional properties of pharmaceutical interest. The first category of additional components comprises diluents, excipients, sugars, stabilizing agents, labelling agents, organic solvents, additives, preservatives, and/or adjuvants, for instance, those described in WO 2017/085228, WO 2018/210439, WO 2020/104628, or in the literature, e.g., in Remington's Pharmaceutical Sciences (edited by Adeboye Adejare; 23rd edition, 2020). The second category of additional components comprises drugs that can provide additional or synergic activities in combination with the BO-11XSQ formulations, comprising an anesthetic solution that can improve acceptance of the injection or the infusion, and/or pharmaceutical compositions comprising a variety of molecules such as antibodies, hormones, peptides, inhibitors of an enzymatic activity, chemotherapeutic agents, antibiotics, a vaccine (e.g., a vaccine wherein the immunogenic component can be in the form of proteins, peptides, DNA, mRNA, synthetic nucleic acids, replicating or non-replicating nucleic acid vectors, virus-like particles, or inactivated viruses), agonists or antagonists of a receptor or other signaling molecule, and in general any other compound that is described in the literature as providing additional or synergistic therapeutic properties to the Poly(I:C) molecules when administered in connection with a given indication, including cancer or for vaccinating against an infectious agent, and that are suitable or compatible to be simultaneously administered as a single formulation.

The modified BO-11XSQ formulations may be prepared immediately prior to administration to a subject or previously prepared and appropriately stored in vials or syringes at a temperature that avoids degradation, aggregation, precipitation, or other undesirable modification of the combined pharmaceutical composition. The modified formulations can be obtained by directly mixing the BO-11XSQ formulation with the additional component or composition, or may be produced by mixing the BO-11X composition with the additional component or composition and then adjusting the pH of the resulting composition using an appropriate deacidifying solution, e.g., using the buffer and the volumes as described above for preparing the described BO-11XSQ formulations. In both cases, the ratio between the amount or the concentration of particles, or of the Poly(I:C) molecules contained in the composition, and the amount or the concentration of the additional component(s) in the composition may be defined according to one or more desired positive effects on the storage or stability prior to use, any synergistic or additive therapeutic efficacy, pharmacokinetics, bioavailability, acceptability of the method or route of administration (e.g., by injection or infusion, at specific body locations, etc.), the specific indication or status (especially in subjects with cancer), the concurrent use of standard-of-care medical treatments, and/or the overall status of the patient.

The BO-11XSQ formulations herein may be used within 1 minute, 2 minutes, 5 minutes, 10 minutes, 15 minutes, 30 minutes, 60 minutes, 120 minutes or more following preparation thereof, preferably using a deacidifying solution and a BO-11X composition. In another embodiment, the BO-11XSQ formulations are used after having been stored at a temperature of 10°C, 5°C, 0°C, -5°C, -10°C, or -20°C, or at temperatures lower than -20°C, for example, up to -70°C. In another embodiment, the BO-11XSQ formulations are distributed as a batch preparation or as one or more aliquots for single use.

In some embodiments, the batch preparation or one or more aliquots are comprised within a kit or kit of parts. In other embodiments, the deacidifying solution and a BO-11X composition may be provided in a kit or kit of parts comprising preparations of both compositions, either as batch preparations or as aliquots for preparing a single dose for injection. In yet other embodiments, the kit may contain either one of the deacidifying solution and a BO-11X composition already aliquoted for injection while the other component is provided as a batch preparation from which the desired amount of solution is added to the BO-11X aliquot prior to administration. In a preferred embodiment, the kit comprises a BO-11X composition already aliquoted for injection and the deacidifying solution is provided as a batch preparation.

The BO-11X composition and the deacidifying solution may be provided in one or more containers, individually or in combination, within a kit or a kit of parts. In some embodiments, the kit or kit of parts may further comprise instructions for preparing and injecting the described BO-11XSQ formulations, wherein the instructions indicate volumes, dilutions, protocols, and the like, materials for preparing and injecting a BO-11XSQ formulation such as syringes, filters, needles, or other devices, and any additional agent, e.g., anesthetics, diluents, adjuvants, excipients, additives, and/or a further pharmaceutical composition for combined administration. The instructions and materials that may be present in the kit can be adapted according to the route of administration and the actual medical use, for instance, according to the type of cancer, immunological or genetic characteristics of the patient to receive the therapeutic composition, and/or the appropriate cancer treatment protocol.

### Methods of Use and Administration of BO-11XSQ Formulations

The present invention further relates to uses and methods applicable to the pharmaceutical compositions disclosed herein, which are based on BO-11X compositions as described in WO 2017/085228, WO 2018/210439, WO 2020/104628, and US Ser. No. 17/744,634, all hereby incorporated by reference with respect to the BO-11X composition components, manufacturing of the BO-11X compositions, biophysical features and biological activities of the BO-11X compositions, and other criteria that specifically apply, or are compatible with, parenteral administration, including administration by injection or infusion, in addition to related regulatory requirements including GMP (Good Manufacturing Practices) guidelines, and other relevant international or national standards for the preparation and administration of drugs. Moreover, the percentages and variable lengths of Poly(C), Poly(I), and Poly(I:C) as described herein for the preparation of BO-11X formulations may be used to define a composition of alternative Poly(I:C)-only formulations that can be administered (e.g., by subcutaneous, intramuscular, or intravenous injection) for treating cancer, alone or in combination with other drugs, vaccines, adjuvants, treatments, and/or any standard-of-care known to be effective, including synergistically, in combination with BO-11X formulations.

According to embodiments of the present invention, specific sizes and concentrations of the Poly(I:C) molecules together with polyethyleneimines bearing certain physiochemical characteristics in the BO-11XSQ formulations allow the administration of the Poly(I:C)-containing particles as compositions providing several advantageous pharmaceutical features. One example of such feature includes producing a robust therapeutic effect, for instance, a durable anti-cancer outcome in a subject injected with the BO-11XSQ formulations, which can further amplify a therapeutic effect of one or more co-administered specific drugs known as having anti-cancer properties. Strategies adopting the BO-11XSQ formulations may be beneficially combined with other standard-of-care cancer therapy regimens that are accepted by medical experts as appropriate courses of treatment for e.g., cancer that are widely used by healthcare professionals. Such regimens may include radiotherapy, cryotherapy, chemotherapy, or tumor ablation and/or anti-cancer drugs. Anti-cancer drugs can be administered, together or sequentially, with standard-of-care therapies, with BO-11XSQ formulations using the same or different modes of administration, e.g., routes of parenteral administration or intratumoral administration, especially in cases where the cancer is metastatic and/or refractory or otherwise resistant to such standard-of-care cancer treatment or to such anti-cancer drugs, i.e., refractory or resistant to immunotherapy involving the administration of an anti-PD-1 or anti-PD-L1 antibody.

The disclosed BO-11XSQ formulations as a combination therapeutic approach with one or more anti-cancer drugs may be administered as two separate injectable pharmaceutical compositions or combined together and then administered as a single injectable composition. When administered separately, the BO-11XSQ formulation and the additional anti-cancer drug can be administered at different times in the same location, in two different locations (e.g., through the skin at two different parts of the body), or by using two different administration modes (e.g., a first mode by subcutaneous, intravenous, or intramuscular injection and a second mode by intratumoral injection).

The therapeutic effects of a BO-11X composition may be defined as reducing the size of a tumor or inhibiting its growth or a reduced resistance and/or increased sensitivity to another cancer therapy, e.g., by overcoming escape mechanisms that may include mutations that alter specific genes, pathways, and/or response to drugs or endogenous compounds such as cytokines. Other therapeutic effects following the administration of the BO-11X compositions may comprise reducing tumor malignancy and metastasis, reduction of the quantity or quality of medical interventions and/or the overall pain level and/or discomfort experienced by a subject following administration. These clinical effects may be evaluated not only at the level of standard technical criteria for evaluating cancer progression or invasiveness, but can also be found associated to, or anticipated by, the detection of changes in biological features that are known to relate to an improved response to the cancer treatment at various clinical stages, which can be determined within the tumor, in the surrounding tissues or organ where the tumor is located, in biological fluids (such as urine or blood), and/or in other tissues. Such detection can be made using conventional diagnostic technologies based on imaging, blood analysis, biopsies, and/or other biomolecular tools.

Depending on the type of cancer to be treated by the disclosed compositions, these biological detection criteria may be changes in the amount of specific cell populations (e.g., immune cells such as antigen-specific CD8+ T cells, dendritic cells, T regulatory cells, T cells and/or NK cells), cytokines (e.g., interferons and/or interleukins), and/or other molecular biomarkers (e.g., growth factors, protein receptors, enzymes, transcription factors and the like) that can be identified on the surface of (or within) cancer cells, in tissues, organs, blood, and/or urine. These criteria may be evaluated by analyzing data obtained by exposing cell preparations, tissues, organs, animals, organoids, human or clinical samples (e.g., biopsies, blood or plasma preparations) or other biological samples or extracts useful for cancer detection and potentially for defining which distinct biological targets and effects are a direct result of, or associated with, the biological activities that BO-11XSQ formulations exert on such cells (e.g., apoptosis, autophagy, activation, proliferation, or other) or such cells exert in human body (e.g., within the tumor, in lymph nodes, in blood and the like).

The biological effects of the BO-11XSQ formulations according to the present invention may be detected and/or defined using a combination of physiological criteria, for instance, observing coordinated or parallel changes in the secretion of chemokines, cytokines, interferons or in the expression of specific cell surface receptor or transcription factors. These observations may be useful for validating the therapeutic use of the BO-11XSQ formulations alone or in a combination therapy that further includes a second therapeutic agent or standard-of-care treatment including radiotherapy, cancer immunotherapy, or tumor ablation. In particular, when compared to clinical or therapeutic effects observed using various cancer experimental models or in patients actually treated (or a potential candidate for treatment) with a BO-11X composition or another drug, the present invention may advantageously permit the identification of novel clinical uses, methods of treatment and drug regimens that can benefit from treatment using the present BO-11XSQ formulations, alone or in combination with other drugs, including anti-cancer drugs (e.g., standard-of-care treatments or antibodies against cancer antigens), either during the course of, or prior to, such treatment.

The BO-11XSQ formulations herein may provide significant improvements for the therapeutic management of cancer. In particular, the BO-11XSQ formulations according to the invention provide a pronounced clinical efficacy with respect to untreated tumor lesions, circulating cancer cells, (micro)metastasis and other damage produced by the persistence of cancer cells in the body of a subject. The administration of a BO-112XSQ formulation as disclosed herein may advantageously reduce the burden, persistence, metastasis, malignancy, and/or recurrence of a tumour that can be identified to a wide variety of cancers, including melanoma, cervical cancer, liver cancer, breast cancer, ovarian cancer, pancreatic cancer, kidney cancer, prostate cancer, testicular cancer, urothelial carcinoma, bladder cancer, non-small/small cell lung cancer, colorectal adenocarcinoma, colorectal cancer, mesothelioma, gastrointestinal stromal tumors, biliary tract cancer, gastroesophageal carcinoma, myelodysplasia syndrome, transitional cell carcinoma, neuroblastoma, Wilms tumor, brain cancer, colon cancer, head/neck squamous cell carcinoma, hepatocellular carcinoma, and/or any other carcinoma or sarcoma. Exemplary cancers contemplated for treatment by the BO-11XSQ formulations are refractory cancers or cancers that are resistant to radiotherapy, chemotherapy or immunotherapy, such as colorectal or gastric cancer, Non-Small-Cell Lung Carcinoma, liver cancer, soft tissue sarcoma, or malignant melanoma, and other solid tumors.

### Dosage and Regimens of BO-11XSQ Formulations

The dosages, volumes, and regimens for administering a BO-11XSQ formulation according to the present invention may be defined and optimized on the basis of known properties of the BO-11X formulations, in particular as described WO 2017/085228 and WO 2018/210439, incorporated herein by reference. Moreover, WO 2020/104628, also incorporated herein by reference, and other disclosures reporting clinical studies involving BO-112 formulations or its labelled derivatives (e.g., BO-11XL formulations) may be exploited to additionally adjust the dosage, the volume, the regimen or mode of administration, the selection of patients to be treated, any additional medical interventions (e.g., surgery or other tumour ablation techniques), and the appropriate clinical criteria useful for assessing the outcome of BO-11XSQ administration, alone or in combination with the administration of another pharmaceutical composition.

A suitable dosage of a BO-11XSQ formulation that can be administered by means of a single injection may be determined according to the amount of Poly(I:C) molecules contained therein, for instance, between 0.01 mg and 5000 mg, including any intermediate value, for example, 0.05, 0.1, 0.5, 1.0, 2, 5, 10, 20, 50, 100, 250, or 500 mg, preferably, from about 0.5 mg to about 2.5 mg of Poly(I:C) molecules. The amount of Poly(I:C) molecules may be also determined according to the body weight of the subject, for instance between about 0.01 mg/kg to about 20 mg/kg of body weight, between about 0.05 mg/kg to about 10 mg/kg or between about 0.01 mg/kg to about 1 mg/kg of body weight. Alternatively, the amount of Poly(I:C) molecules may be also determined according to a period of time during which the BO-11XSQ formulation is to be administered (e.g., per day, per week, per month, or during each cycle of treatment), for instance, about 0.01 mg/day to about 20 mg/day, between about 0.05 mg/day to about 10 mg/day or between about 0.01 mg/day to about 1 mg/kg of day. The corresponding volume of a BO-11XSQ formulation to be administered by means of a single injection or infusion can be determined accordingly, and depending on the dilution of initial BO-11X composition prior to the addition of the deacidifying solution, can be a volume of between 0.1 and 50 ml, preferably between 1 ml and 25 ml, such as 1 ml, 2 ml, 3 ml, 5 ml, 10 ml, 12 ml, 15 ml, 20 ml, or 25 ml.

The term "about" as used herein, when referring to a measurable value such as a parameter, an amount, and the like, is intended to encompass a degree of variability in a value or range, for instance, a variability of +/-20% or less, preferably +/-10% or less, more preferably +/-5% or less, even more preferably +/-1% or less, and still more preferably +/-0.1% or less of and from the specified value. Such variations are appropriate to perform the disclosed invention.

The location, number, and frequency for administering a BO-11XSQ formulation herein may be defined according to the specific therapeutic needs and cancer status of the subject in need of such treatment, with two or more injections that may be performed within a predefined number of hours, days, or weeks, and in, e.g., two, three, or more cycles of treatment. When injection of a BO-11XSQ formulation is combined with a second treatment, which may include combining with an intratumoral administration of a BO-11X formulation or of a pharmaceutical composition comprising another anti-cancer agent, the two treatments within one or more minutes, in any order) or sequentially (administered as two or more distinct medical acts separated by hours, days, or weeks), with or without the concurrent use of standard-of-care treatments. The combined administration of a BO-11XSQ formulation and of a second pharmaceutical composition (which can be an intratumoral administration of a BO-11X formulation), may be adapted to other cancer treatment protocols and drug regimens that are administered (simultaneously or sequentially) using the same route or alternatively, using other routes and means, including by infusion, orally, subcutaneously, intradermally, intranasally, intravenously, intramuscularly, intrathecally, intranasally, intravesically, intratumorally, intra-articularly, topically, and transdermally. Lists and standard definitions of these and other clinically acceptable routes of administration can be found in the regulatory documentation provided by authorities including the Food and Drug Administration (FDA; CDER Data Element Number C-DRG-00301, Data Element Name: Route of Administration).

The volume, dosage, and regimen for administering a BO-11XSQ formulation in combination with another drug (or the combined intratumoral administration of a BO-11X formulation) according to the present invention may be defined on the basis of the protocols described for a BO-11X formulation in the literature, for instance, as described in WO 2017/085228, WO 2018/210439 and WO 2020/104628. Alternatively, the amount of either BO-11XSQ formulation in combination with another drug (or the combined intratumoral administration of a BO-11X) can be defined as the total amount to be injected in two or more injections during a single or multiple cycles or treatment (e.g., on a daily, weekly, or monthly basis). The ratio between the amount of the two therapeutic agents to be administered may be adapted, for instance, by reducing the concentration of either agent in order to validate the minimal amount of the agent that is required to obtain the desired synergistic, or additional, therapeutic effect on a tumor, related lesions and/or metastasis, by performing the lowest possible number of injections (or other therapeutic intervention) and by controlling for any undesirable effect, poor compliance, or unsatisfactory response to the treatment. The volume of the BO-11XSQ formulation (which may comprise a second therapeutic agent) for each injection can be adjusted accordingly (e.g., a volume of 0.1 ml, 0.5 ml, 1.0 ml, 1.5 ml, 3 ml, 5 ml, 7.0 ml, 10.0 ml, or more per injection) to provide each respective agent in an appropriate amount and concentration, which may be further adapted during each cycle of treatment using conventional techniques as needed.

A BO-11XSQ formulation according to the invention, e.g., a BO-112SQ formulation, may be presented in a unit dosage form and may be prepared by using known methods for intratumoral or other parenteral, injection-based administration. Such methods generally involve bringing one or more therapeutic agent into association with a carrier, which can include one or more accessory ingredients and can also vary according to the contemplated method of treatment, the dosage form, and the mode of administration. Numerous factors may modify the physiological action of a BO-11XSQ formulation (e.g., a BO-112SQ formulation) including body weight, gender, diet, time of administration, rate of excretion, condition of the subject, drug combinations, genetic disposition, and reaction sensitivities, which are known to those skilled in the art. Administration of the instant formulations may be carried out continuously or in one or more discrete doses within a maximum tolerated dose, adapting, as needed, any other drug or standard-of-care treatment.

The administration of a BO-11XSQ formulation according to the present invention may be employed as a rescuing, sensitizing, or as a maintenance combination treatment that can improve the efficacy, safety, and/or clinical use of other drugs and treatments disclosed herein, such as surgery, radiotherapy, chemotherapy, toxin therapy, cancer vaccination, laser therapy, phototherapy, immunotherapy, cryotherapy, cell-based therapy, or gene therapy. Preferably, a combination approach comprises the administration of agents useful for cancer immunotherapies, for instance, an anti-PD-1 antibody or an anti-PD-L1 antibody (such as pembrolizumab or nivolumab). In particular, the therapeutic approaches described herein comprising the administration of a BO-11XSQ formulation advantageously provide effects for treating cancers that are refractory or resistant to conventional treatments such as radiotherapy, chemotherapy or immunotherapy or other treatment strategies involving surgical excision, thermal or electrical ablation, cryotherapy, laser therapy, or phototherapy more effectively and/or in a more acceptable and beneficial manner for the patient. Moreover, the present invention also relates to therapeutic, anti-cancer effects provided by the administration of a BO-11XSQ formulation that are not observed when a BO-11X formulation is administered by intertumoral injection, or when the intertumoral injection of the BO-11X formulation may not be technically feasible.

The present invention further relates to methods of treatment and other medical regimens that may benefit from the administration of a Poly(I:C)-containing composition, such as a BO-11X formulation and in particular a BO-112 formulation, in a preparation format that is well-suited for injection or general parenteral administration because it presents a pH closer to a standard physiological range. The methods of treatment and medical regimens involving the administration of a BO-11XSQ formulation ,and in particular a BO-112SQ formulation, as described herein may be suitable for a given cancer (i.e., a specific type of cancer according to the presenting pathophysiology, metastatic properties, location, stage, etc.), populations of candidate patients (i.e., defined by prior treatments, ongoing standard-of-care or other treatment, immunological profile, altered gene copy number, or genetic mutations or activation of relevant genes), and/or in combination with one or more additional compounds having therapeutic or prophylactic properties (e.g., checkpoint inhibitors, antibodies targeting cancer antigens, cell-, peptide- or nucleic acid- based cancer vaccines, non-human antigens for vaccination against infective agents, or adoptive cell-based therapies). In addition, the comparison of effects exerted by a BO-11XSQ formulation such as a BO-112SQ formulation, with other such therapeutic compounds in clinically relevant models, can advantageously permit the identification of patients, or sub-populations of patients, the immunological profile, and/or the cancer stage or type, which may positively respond to a treatment protocol based on the present BO-11XSQ formulations such as a BO-112SQ formulation, combined with the administration of one or more therapeutic or prophylactic compounds. These alternative, novel therapeutic drug regimens may involve the administration of BO-11XSQ formulations, such as a BO-112SQ formulation, within a tissue, an organ, and/or through skin (e.g., by subcutaneous, intravenous, or intramuscular injection) combined with the administration of a second agent at the same location (or using the same route of administration) or in another location (or using a different route of administration), in particular at a specific pathological altered locations such as a tumor or related cancer lesions.

The route and frequency of administering the BO-11XSQ formulations according to the invention, such as a BO-112SQ formulation, and/or of administering the second agent can be determined by a clinician, for example, in accordance with the applicable clinical parameters, i.e., a response defined by analyzing relevant biomarkers, gene expression signatures, cancer antigens, immune cells, or other clinical criteria (e.g., tumor burden, stage of the tumor, amount of metastasis, and/or tumor recurrence). Moreover, the various regulatory institutions responsible for the evaluation and/or control of clinical therapeutics (e.g., FDA, EMA, WHO and ICLIO) provide the relevant guidelines and other criteria for evaluating a suitable response to a drug treatment in cancer patients, in general or for specific drugs (e.g. immunotherapies), specific technologies (e.g. PET and imaging), specific biomarkers, and/or specific types of cancers (Liberini V et al, 2021; Lang D et al 2020; van de Donk P et al., 2020).

For example, patients that are resistant or insensitive to an anti-cancer agent (in general or only at level of a specific tumor subtype identified in these patients) may be preferably treated by one or more injections of a BO-11XSQ formulation, such as a BO-112SQ formulation, in which a second agent is also present and co-administered (thereby reducing the number of medical interventions) or by administering the BO-11XSQ and the second agent separately. In one embodiment, such combination approach may involve the administration of one or more anti-PD-1 and anti-PD-L1 antibodies (anti-PD-1/PD-L1) whose efficacy as a cancer immunotherapeutic agent may be improved (or simply made possible) by modifying an existing regimen involving the use of these drugs (involving regular intravenous injections of the antibody) by including one or more injections of a BO-11XSQ formulation (such as a BO-112SQ formulation), for instance, according to the data shown in WO 2017/085228, WO 2018/210439, and WO 2020/104628. This regimen may be initiated either before the beginning of a standard anti-PD-1/PD-L1 clinical protocol or concomitantly with the anti-PD-1/PD-L1 protocol, for instance, by administering the anti-PD-1/PD-L1 agent either every 4, every 3, or every 2 weeks. The injections of the BO-11XSQ formulation (such as a BO-112SQ formulation) may be performed every week or on a daily basis when the anti-PD-1/PD-L1 antibody is also administered.

Alternatively, the drug regimen may include specific weeks wherein only either injections of the BO-11XSQ formulation (such as a BO-112SQ formulation) or anti-PD-1/PD-L1 administration (e.g., by intravenous injection) is performed. This drug regimen may also include a maintenance cycle wherein either the injections of the BO-11XSQ formulation (such as a BO-112SQ formulation) or other drug administration are alternatively or concomitantly administered over a number of weeks, for instance, 2, 4, 6, 8, 10, 12, or more weeks, and such cycle is repeated 2, 3, 4 or more times. The drug regimen may further comprise the injections of the BO-11XSQ formulation (such as a BO-112SQ formulation) at least two, three, four, five, six, seven, eight, nine or ten times in each cycle. The injections of the BO-11XSQ formulation (e.g., a BO-112SQ formulation) in each cycle may be separated by a predefined number of hours, for example, 1, 3, 6, 12, 18, 24, 36 or more hours, or by a predefined number of days, for instance, 2, 3, 5, 7, 10 or more days, over a period of 1, 2, 3, 4 or more weeks.

A cancer patient may be identified as a suitable candidate for treatment with the BO-11XSQ formulation (such as a BO-112SQ formulation) according to the present invention, as a first line, second line, or later line of treatment, by presenting the appropriate combination of clinical parameters and/or biomarkers and/or not responding to standard-of-care cancer therapies (such as radiotherapy or chemotherapy) or drugs that target to specific cancer antigens, immune checkpoints, cells, or biological pathways. These drugs may have a different chemical nature. In some embodiments, the drug can be a small molecule (e.g., an inhibitor of kinases or other enzymes), a peptide (e.g., a cancer vaccine), an antibody (e.g. directed against PD-1, PD-L1, CTLA4, or CD20), a cell-based products (e.g., adoptive cell transfer as in adoptive T cell therapy and adoptive immunotherapies in general), or a nucleic acid (e.g., a modified nucleotide, small interfering RNA, aptamer, an oligonucleotide, mRNA, an antisense nucleic acid, targeting TLR9 or other receptors such as STING, replicating or non-replicating DNA vectors encoding specific cancer antigens or therapeutic proteins). With respect to antibodies directed to cancer antigens, exemplary antigens are those approved (or under validation and review) by regulatory authorities including the EMA (in Europe) or the FDA (in the USA), in addition to targeting cell surface proteins, in particular for treating solid tumors, and characterized by a therapeutic activity and/or a clinical use that may be improved by a combined administration with a pharmaceutical composition having immunostimulatory activities, including one or more injections of a BO-11XSQ formulation (such as a BO-112SQ formulation). Exemplary antigens include PDGFR, Her2, EGFR, VEGFR2, EpCAM, CD40, and CD25 (Zahavi D and Weiner L, 2020; Xiong H et al., 2021).

The methods of treatment and uses according to the present invention relate to treating or preventing a cell growth disorder characterized by abnormal growth of human or animal cells, for instance, due to cancer (that is, involving tumorigenic transformation, metastasis, and/or any tumorigenic compound). Exemplary models for evaluating the methods or treatment and uses of the present invention are described in WO 2017/085228, WO 2018/210439, WO 2020/104628, and in the literature describing the clinical studies in which BO-112 formulation (an exemplary BO-11X formulation) has been administered alone or in combination with other treatments (such as anti-PD-1 antibodies or radiotherapy). Preferably, the methods of treatment and uses of the present invention are intended for inducing (directly or indirectly) the death of one or more tumor cells or suppressing growth of the one or more tumor cells, and further including treating, reducing, ameliorating, or preventing cancer growth (in particular to obtain permanent growth arrest or senescence of the tumor and cancer cells), survival, metastasis, epithelial-mesenchymal transition, immunologic escape or recurrence. In some embodiments, the BO-11XSQ formulation (such as a BO-112SQ formulation) is used to treat cancers at various stages (e.g., Stage I, or Stage II, or Stage III, or Stage IV). By way of a non-limiting example, using the overall stage grouping, Stage I cancers are localized to one part of the body; Stage II cancers are locally advanced, as are Stage III cancers. Whether a cancer is designated as Stage II or Stage III usually depends on the specific type of cancer. In one non-limiting example, Hodgkin's disease, Stage II indicates affected lymph nodes on only one side of the diaphragm, whereas Stage III indicates affected lymph nodes above and below the diaphragm. The specific criteria for Stages II and III therefore differ according to diagnosis. Stage IV cancers have often metastasized or spread to other organs or throughout the body. Alternatively, the methods of treatment and drug regimens according to the present invention are used for adjuvant therapy, i.e., a treatment that is given in addition to the primary, main or initial treatment. By way of a non-limiting example, adjuvant therapy may be an additional treatment usually given after surgery where all detectable disease (in the form of tumors or other lesions, for instance) has been removed, but where there remains a statistical risk of relapse due to occult disease. In some embodiments, the agents described herein are used as an adjuvant therapy in the treatment of a cancer.

According to the present invention, the cancer being treated by the disclosed compositions, combinations, drug regimens, and related methods of administration, preferably by injection (or infusion), is one or more of following cancers: basal cell carcinoma, biliary tract cancer; bladder cancer; bone cancer; brain and central nervous system cancer; breast cancer; cancer of the peritoneum; choriocarcinoma; connective tissue cancer; cancer of the digestive system (including esophageal, stomach, colon, rectal or other gastrointestinal cancer); eye cancer; cancer of the head and neck; glioblastoma; hepatic carcinoma; hepatoma; intra-epithelial neoplasm; kidney, adrenal, or renal cancer; leukemia; liver cancer; lung cancer (e.g., small-cell lung cancer, non-small cell lung cancer, lung adenocarcinoma, and lung squamous carcinoma); melanoma; renal cell carcinoma; myeloma; neuroblastoma; oral cavity cancer (lip, larynx, tongue, mouth, and pharynx); pancreatic cancer; prostate cancer; retinoblastoma; rhabdomyosarcoma; leiomyosarcoma; cancer of the respiratory system; salivary gland carcinoma; skin cancer; squamous cell cancer; testicular cancer; thyroid cancer; uterine, endometrial, cervical, vulvar, ovarian or other gynecological cancer; cancer of the urinary system; lymphoma including B-cell lymphoma, Hodgkin's and non-Hodgkin's lymphoma (NHL; including specific types such as low grade/follicular, small lymphocytic, intermediate grade/follicular, intermediate grade diffuse, high grade immunoblastic, high grade lymphoblastic, high grade small non- cleaved cell, or bulky disease NHL), mantle cell and AIDS-related lymphoma; chronic lymphocytic leukemia; acute lymphoblastic leukemia; Hairy cell leukemia; chronic myeloblastic leukemia; as well as other carcinomas and sarcomas; post-transplant lymphoproliferative disorder (PTLD), abnormal vascular proliferation associated with phakomatoses or edema (such as those that associated with brain tumors). Additional types and definition can be found in National Cancer Institute website as in the webpage dedicated to cancer types at https://www.cancer.gov/types.

In some embodiments, the cancer is a biliary tract cancer. In further embodiments, the biliary tract cancer is selected from pancreatic cancer, gallbladder cancer, bile duct cancer, and cancer of the ampulla of Vater. In some embodiments, the cancer is liver cancer. In some embodiments, the cancer is colon cancer. In some embodiments, the biliary tract cancer is cholangiocarcinoma and/or an adenocarcinoma. Alternatively, the cancer may be listed among the list of rare diseases, being defined according to the criteria of incidence and/or prevalence as defined in Europe or USA and indicated in the regularly updated lists made available in the website of organizations such as the International Rare Cancers Initiative (https://project.eortc.org/irci/).

More preferably, a BO-11XSQ formulation (e.g., a BO-112SQ formulation) according to the invention is used in methods of treatment for treating solid tumors, such as carcinomas, gliomas, melanomas, or sarcomas. The BO-11XSQ formulation (such as a BO-112SQ formulation) described herein may be administered by subcutaneous, intravenous, or intramuscular injection or administered directly within or at a location proximal to the tumor, for example, at the margin of the tumor mass, in the surrounding epithelial cells, lymphatic and/or blood vessels including by intratumoral or peritumoral injection. The cancer may be a dormant tumor, which may result from the metastasis of a cancer. The dormant tumor may also be left over from surgical resection or removal of a tumor. The cancer recurrence may, for example, be tumor regrowth, a lung metastasis, or a liver metastasis, wherein the methods of treatments and uses of the invention may reduce or block metastasis in distant sites that tumor would have caused.

Additionally, macroscopic examination of organs and skin and microscopic, pathological analysis in either immune-deficient fully immune competent animal models may further indicate the efficacy of the methods and uses according to the present invention. The quantitative data that are generated in similar studies can be compared among the different experimental groups by using the appropriate statistical tests, with and without corrections for multiple testing, at the scope to evaluate which therapeutic (in particular anti-tumor) effects are provided by the administration of a BO-11XSQ formulation (e.g., a BO-112SQ formulation), alone or in combination with any other drug or treatment. Moreover, the methods of treatment and drug regimens according to the present invention may improve the effect of a BO-11XSQ formulation (e.g., a BO-112SQ formulation), in inducing local or systemic immunity by promoting immune cell memory allowing its use as single therapeutic agent, or in combination with immunomodulatory compounds, tumor-targeting agents, oncolytic viruses, adoptive cell transfer and other cell-based therapies, DNA- or peptide-based vaccines, or inhibitors of immunosuppressive or other cancer-related metabolic pathway.

Furthermore, the disclosed compositions can be administered for supporting vaccines, cytokines, antigens, antibodies, chemical compounds, and other compounds having immunomodulatory activities for treating or preventing cancers (solid or not) or infection, for instance as adjuvant and/or for rescuing patients poorly responding or resistant to a drug, including agents for cancer immunotherapy, for altering cell metabolism and/or functions (preferably, in immune and/or cancer cells), for modulating DNA expression, replication and/or repair (including drugs that target epigenetic mechanisms), or for standard-of-care therapies (such as chemo- or radiotherapy, or vaccine-based therapies involving cancer or viral antigens). Such additional therapeutic agent (in the form of protein or corresponding mRNA) that is co-administered (subsequently, in any order) with a BO-11XSQ formulation (such as a BO-112SQ formulation), may improve a method of treatment with respect to the bioavailability, efficacy, pharmacokinetic and/or pharmacodynamic profiles, stability, metabolization, or other pharmaceutical property compared to the case when treatment with a BO-11XSQ formulation (such as a BO-112SQ formulation), or another compound of pharmaceutical interest is administered alone.

The methods of treatment and regimens of the present invention may additionally include the administration of an immune-modulating agent that is preferably an antibody including a monoclonal antibody and other antibody formats, or any other pharmaceutically available agent that binds a cell surface protein that control immune response, thus acting as a checkpoint inhibitor (CPI), which can block, reduce and/or inhibit PD-1 and PD-L1 or PD-L2 and/or the binding of PD-1 with PD-L1 or PD-L2. Alternatively, the CPI can block, reduce and/or inhibit the activity of other immune checkpoint molecules such as LAG3, ICOS, CD137, CTLA-4, AP2M1, LAG3, OX-40, CD80, CD86, SHP-2, and/or PPP2R5A. As a further alternative, the CPI increases and/or stimulates CD137 (4-1BB) and/or the binding of CD137 (4-1BB) with one or more of a 4-1BB ligand and TRAF2. Other examples of a second therapeutic agent having immunomodulating properties include radiotherapy, chemotherapy CAR-T cells, cancer antigen vaccines, or agents that target regulatory T cells, metabolic enzymes, DNA repair and/or replication. The present invention also provides combining a BO-11XSQ formulation (such as a BO-112SQ formulation), with an immunomodulatory agent and/or with one or more common cancer treatment regimens (e.g., FOLFOX, FOLFIRI, radiation, photodynamic therapy or an antiproliferative agent such as doxorubicin, daunorubicin, mitomycin, actinomycin D, bleomycin, cisplatin, VP16, enedyine, vincristine, vinblastine, carmustine, a STING agonist, and the like).

Exemplary cancer indications wherein the methods of treatment and drug regimens according to the present invention can be pursued by appropriately combining the administration of an agent inhibiting PD-1 (such an anti-PD-1 antibody) with or without a standard-of care treatment (for instance, radiotherapy, as an adjuvant), include, but are not limited to, melanoma, triple negative breast cancer, sarcoma, head-and-neck cancer, colorectal cancer, bladder cancer, renal cell carcinoma, liver metastasis, gastric cancer, prostate cancer, and hepatocellular carcinoma. Such indications may be also treated by administering a BO-11XSQ formulation (such as a BO-112SQ formulation), with an anti-PD-1, anti-PD- L1, an anti-CTLA4, or an anti-OX-40 antibody using an optimized drug regimen design. In some embodiments, the immune-modulating agent targets one or more of PD-1, PD-L1, and PD-L2. Preferably, the immune-modulating agent is a PD-1 inhibitor. In some embodiments, the immune-modulating agent is an antibody specific for one or more of PD-1, PD-L1, and PD-L2. Such immune-modulating agent is an antibody, including the non-limiting examples of nivolumab (ONO-4538/BMS-936558, MDX1106, OPDIVO), pembrolizumab (KEYTRUDA), pidilizumab (CT-011), MK-3475, BMS 936559, MPDL3280A. For example, the BO-11XSQ formulation (such as a BO-112SQ formulation) is combined with one or more of MPDL3280A (optionally with vemurafenib) and MEDI4736 (optionally with one or more of dabrafenib and trametinib) for the treatment of melanoma. Alternatively, as also described in WO 2018/210439 in connection with the described BO-11X formulations, a PARP inhibitor may be used in combination, or administered with, a BO-11XSQ formulation in patients where PARP inhibitors (PARPi) were observed to be partially or completely ineffective, perhaps requiring sensitization for eliciting a response. Certain PARPi compounds that have received official marketing approval (e.g., Olaparib, Rucaparib, Niraparib, Talazoparib, and Pamiparib), along with their clinical applications (e.g., in particular for treating ovarian, prostate, or breast cancer), and observed resistance mechanisms shown to reduce PARPi efficacy have been recently reviewed (Fu X et al., 2023). The presently described BO-11SQ formulations may improve the outcomes of cancer therapeutic protocols based upon PARPi administration.

More particularly, a BO-11XSQ formulation (such as a BO-112SQ formulation), disclosed herein may be used for obtaining a synergistic therapeutic effect when administered with the second therapeutic agent, including reducing the regular dosage and/or frequency of administration of the second therapeutic agent (thus potentially reducing the need for additional medical intervention, an undesirable resistance to drugs, and/or a patient's overall discomfort). Moreover, the present BO-11XSQ formulations (such as a BO-112SQ formulation), when administered with the second therapeutic agent, may allow for treating patients that are resistant, insensitive, or presenting a poor clinical response to the second therapeutic agent, by overcoming any specific tumor resistance or escape mechanism (including mutations that alter specific genes, pathways, and/or response to drugs or endogenous compounds such as cytokines). Thus, the BO-11XSQ formulations disclosed herein (such as a BO-112SQ formulation) may be used as a drug-rescuing or drug-sensitizing combination treatment, preferably for the treatment of cancer. Such methods of treatment may involve administering a BO-11XSQ formulation (such as a BO-112SQ formulation), by parenteral injection (e.g., subcutaneously, intramuscularly, or intravenously) and/or by intratumoral or peritumoral injection (within the tumor, at the margin of the tumor mass, in the surrounding epithelial cells, lymphatic or blood vessels). Alternatively, other means may allow administering a BO-11XSQ formulation (such as a BO-112SQ formulation), directly within or in the proximity of cancer cells or organ comprising the cancer cells, and the systemic administration of an immunostimulatory agent.

The present invention also provides methods of treatment and drug regimens involving the administration of a BO-11XSQ formulation (such as a BO-112SQ formulation) for increasing an immune response against a pathogen or other undesirable biological agent, and in particular for enhancing an anti-tumor immune response, potentially itself acting as an immune-modulating agent. Such an effect may be monitored by measuring a tumor-related immune response at the tumor site and tumor microenvironment (or in the bloodstream, other biological fluids, and tissues) at the level of relevant cell types or subpopulations (e.g., dendritic cells, T regulatory cells, T cells and/or NK cells) and/or of immunological biomarkers (e.g., chemokines, growth factors, cytokines, and their receptors). If a BO-11XSQ formulation (such as a BO-112SQ formulation) is clinically administered by subcutaneous, intramuscular, intravenous or intratumoral injection, apoptosis and/or necrosis is observed in the tumor, thus potentially promoting the presentation of tumor antigens to resident dendritic cells. In the domain of cancer immunotherapy, a BO-11XSQ formulation may be administered in combination with immune checkpoint blockades (ICBs), adoptive cell transfer therapies (ACTs), or replicating or non-replicating viral vectors that permit the expression of cancer immunogenic antigens or neoantigens, or dendritic cell (DC)-based cancer vaccines that are modified *ex vivo* and are generated by continuously evolving technologies (Lee K et al., 2023). A signaling cascade may lead also to the recruitment of immune cells, in particular CD4+ and CD8+ T cells into the tumor mass promoting an immune effect against the tumor, contributing to the cytotoxic effects of a BO-11XSQ formulation (such as a BO-112SQ formulation). Otherwise, the administration of a BO-11XSQ formulation (such as a BO-112SQ formulation), may induce changes in the absolute value and/or in the ratio of specific immune cell populations within a tumor, lymph nodes, or both.

The analysis of the biological samples that are obtained from subjects treated with a BO-11XSQ formulation to establish the clinical status and response of the subject to the dose and regimen for a given pathology, may involve the use of multiple approaches for detecting and quantifying changes in the subject, including medical imaging, for instance, radiological techniques, nuclear magnetic resonance, or echography, or bioanalytical methods as applied within cells, tissues, or biological fluids (e.g., biopsies, blood analysis, or urine analysis). The applicable guidelines, clinical protocols, and medical literature regarding the diagnosis and/or treatment of a specific disease can be used to select and compare the appropriate biomarkers, imaging techniques, and bioanalytical methods to be applied for establishing the route, dosage, and/or regimen for achieving an optimized response and administration of a BO-11XSQ formulation.

In parallel to the disease-specific evaluations described herein, it may be also relevant to apply methods for evaluating the presence of the particles comprised in a BO-11XSQ formulation within the same (or different) biological samples to evaluate where and for how long these particles (or at least the non-natural Poly(I:C) molecules within them) are circulating in the body of the subject within hours, days, or weeks after administration. The methods for quantifying Poly(I:C) molecules as a surrogate marker for the presence and/or activity of the particles of BO-11XSQ formulation in biological samples (in particular blood, serum, or plasma of the subject) may involve the use of antibodies that are directed against Poly(I:C) molecules or at least against double-stranded RNA (dsRNA) components present within the Poly(I:C) molecules. A series of monoclonal or polyclonal anti-dsRNA antibodies have been generated in different model animals (rat, rabbit, mouse, etc.) and their use has been widely reported in the literature. Some of the more commonly used anti-dsRNA antibodies are identified as J1, J2, J5, K1, K2, and are available from various commercial providers (e.g., Jena Biosciences, Sigma-Aldrich, Abcam, or Novus Biologicals) and may be integrated into kits and protocols such as those for performing ELISA (enzyme-linked immunosorbent assay) or other antibody-based assays in a solid or liquid phase.

Preferably, such analytical methods involve exposing the biological sample to an agent that can facilitate the interactions between the anti-dsRNA antibody and the Poly(I:C) molecules by, for example, separating the Poly(I:C) molecules from the synthetic polymers forming the particles that are present in the sample. This preliminary step may involve exposing the biological samples to one or more agents that partially denature, dissociate, or disaggregate the particles in a liquid sample. A first category of such agents includes heat, but this step may be more precisely controlled using compounds that influence electrostatic interactions between the Poly(I:C) molecules and the synthetic polymers and/or compounds that influence hydrophobic interactions within macromolecules by, e.g., disrupting hydrogen bonding. In the former case, the charged groups present in carbohydrates, polysaccharides, glycosaminoglycans, or other synthetic or natural polymers may exert sufficient electrostatic forces such as those exerted by heparin, an exemplary agent for performing this process. In the latter case, the compound may include a molecule that is conventionally defined as a chaotropic agent, e.g., n-butanol, ethanol, guanidinium chloride, lithium perchlorate, lithium acetate, phenol, 2-propanol, thiourea, and urea, preferably guanidinium chloride and/or urea. Both categories of compounds are commercially available and may be added alone or in combination to a solution useful for diluting the biological sample, such as blood or a specific fraction (e.g., plasma), in an amount sufficient for separating the Poly(I:C) molecules within the particles in suspension, with or without applying heat in addition.

The Poly(I:C) molecules that are released, and the dsRNA regions present within these molecules, are then detected and preferably immobilized on beads, plates, or other appropriate solid supports by adding a first anti-dsRNA antibody to the biological sample. The quantification of antibody-bound Poly(I:C) molecules may be performed by using a labeled compound (e.g., a fluorescent, radioactive, luminescent, metabolic, or other chemically or enzymatically detectable label) that binds the first anti-dsRNA antibody or, alternatively, by adding a further anti-dsRNA antibody that allows further amplifying the signal obtained using the labeled compound.

The bioanalytical methods useful for detecting and quantifying the Poly(I:C) molecules may also provide a specific readout for determining the lower limit of quantification (LLOQ) and the upper limit of quantification (ULOQ) that provides a satisfactory level of accuracy, precision, linearity of the calibration standards, and other relevant criteria that affords the standardized use of such methods in the clinical practice. The range of quantification may be defined between 0.1 ng/ml (as the LLOQ value) and 500 ng/ml (as the ULOQ value), preferably between 1 ng/ml (LLOQ) and 100 ng/ml (ULOQ), more preferably between 1 ng/ml (LLOQ) and 25 ng/ml (ULOQ). The sensitivity and specificity of the assay employed for measuring the Poly(I:C) molecules may be improved and adapted to specific types of biological samples not only by following the guidance and instructions of the materials and equipment for performing the assay, but also by taking into account the official requirements and recommendations according to the guidelines for bioanalytical methods applicable to chemical and biological drugs as issued by the pertinent regulatory authorities, medical associations, or other institutions (Kaza M et al., 2019; Sankar P et al., 2019). Relevant documentation is available from the Food and Drug Administration ("FDA," see in particular "Bioanalytical Method Validation - Guidance for Industry", May 2018, docket no. FDA-2013-D-1020), the European Medicines Agency ("EMA", see in particular EMEA/CHMP/EWP/192217/2009 and the ICH Harmonized Tripartite Guideline: Validation of Analytical Procedures, CPMP/ICH/381/95, ICH Q2-R1), or the Organization for Economic Co-operation and Development ("OECD"; see in particular in OECD Principles of the of Good Laboratory Practice, GLP).

### EXAMPLES

### Example 1: Selection of a Buffer for Obtaining BO-11XSQ Formulations

### Materials & Methods

The vials of BO-112 composition for preclinical and clinical usages are produced according to the literature (WO 2017/085228 and WO 2018/210439) as a nanoparticle suspension formed with Poly(I:C) molecules and polyethyleneimine (PEl) in a 5% glucose solution. The final formulation is a sterile isosmotic nanoparticle suspension for injection featuring a nominal concentration of 0.6 mg/ml Poly (I:C) molecules on a dry basis and provided in a vial containing 12 ml of the BO-112 composition.

The following buffer solutions were evaluated: sodium hydroxide (NaOH; Sigma-Aldrich, Cat. No. 79724); citrate-phosphate buffer (0.1M citric acid + 0.2M dibasic sodium phosphate; citric acid from Fluka, Cat. No. 27488; dibasic Sodium Phosphate, Na₂HPO₄, from VWR, Cat. No. 28026.260), dipotassium phosphate (K₂HPO₄; Sigma-Aldrich, Cat. No. P8584), sodium phosphate (NaH₂PO₄; Fresenius Kabi, Cat. No. 600210), and sodium bicarbonate (1M or 1/6M NaHCO₃ solution as available from Fresenius Kabi or from B. Braun). The concentration and the pH of the buffer solutions were tested by adjusting the pH of BO-112 composition to more physiological values (pH ≥ 5.0) as shown in Table 1.

**TABLE 1**

| **Buffer Solution** | **Concentration** | **pH** |
|---|---|---|
| Sodium hydroxide (NaOH) | 1M | 14 |
| Citrate-phosphate buffer (0.1 M citric Acid + 0.2 M dibasic sodium phosphate) | 0.1M - 0.2M | 7.6 |
| Dipotassium phosphate (K₂HPO₄)^{∗} | 1M | 9.34 |
| Sodium phosphate (NaH₂PO₄)^{∗} | 1M | 4.31 |
| Sodium bicarbonate (NaHCO₃)^{∗} | 1M (8.4%) or 1/6M (1.4%) | 8.04-8.07 |

| | | |
|---|---|---|
| * Commercially available buffers for intravenous infusion in the clinical setting | | |

Different volumes of room temperature-equilibrated buffer solutions were added to the BO-112 vials (12 ml) using a p200 pipette (PIPETMAN Classic P200; Gilson, Cat. No. F123601) or with a p1000 pipette (PIPETMAN Classic P1000; Gilson, Cat. No. F123602), and then mixed gently using a sideways motion and by gently inverting the vials approximately 10 times to ensure homogenization. Alternatively, the solutions were added to 2 mL of BO-112 composition previously disposed into a 15 ml Falcon tube. Next, 2 ml aliquots of BO-112 composition (deacidified with the different buffers or control/without buffer) were extracted using a 5 ml syringe (BD^{®} Plastipak^{™} Syringe, 3-Piece; Becton Dickinson, Cat. No. 309649) attached to a 20G needle (0.9 x 25 mm; Becton Dickinson, Cat. No. 304827) and filtered with a 0.45 µm cellulose acetate filter (Minisart syringe filters, 28mm diameter; Sartorius, Cat. No. 16555-GUK).

The pH values of the BO-112 composition samples were measured with a pH meter (SevenCompact^{™} Duo; Mettler Toledo, Cat.No. S213) using unfiltered samples as a control. Samples were analyzed by UV-VIS (Ultraviolet-Visible) either pre-filtrated or immediately after the filtration with 0.45 µm cellulose acetate filters. The UV absorbance at 260 nm (A₂₆₀) and at 400 nm (A₄₀₀) was measured (NanoDrop^{™} One; Thermo Scientific, Cat.No. ND-ONE-W) with 2 µl aliquots of control BO-112 composition (unfiltered or untreated) or test samples, using water as a reference.

### Results

The particles in the BO-112 composition display a general tendency to interact electrostatically and/or to form aggregates due to attraction/repulsion forces existing between PEI and Poly(I:C) molecules, i.e., the components of particles comprised in the suspension. A small fraction of such particles is in the near-visual (80-100 microns) and visual (100-250 µm) range and can be filtered with a 0.45 µm filter prior to administration. The removal of visual and near-visual range particles by filtration does not cause any change in the potency of the BO-112 compositions due to a minimal impact that these particles have on the total formulation, when maintained at low pH values (^{~} pH 3). However, an increase of the pH in the formulation by adding a basic solution can cause an alteration of the particle dynamics and aggregation, with the consequential undesirable modification of their size within the BO-112 composition prior to administration.

The *in vitro* comparative analysis of buffers that are suitable for deacidifying pharmaceutical formulations prior to administration were performed by considering the main biophysical acceptance criteria for the BO-112 composition, as determined with or without filtration (the pore size was 0.22 µm or 0.45 µm) to confirm that the particle size and structure is not compromised by the deacidification process. The amount of Poly(I:C) molecules in the BO-112 composition is established by measuring A₂₆₀ value and using, as reference for acceptable loss of material after deacidification, a value lower than 10%. The tendency to form aggregates is established by measuring the A₄₀₀ value using, as a reference for acceptable value after deacidification, a value lower than 0.25. The experiment was performed by using concentrated buffer solutions at high (basic) pH that can be added in very small volumes relative to the BO-112 composition to avoid dilution. The details of this comparative analysis, indicating the percentage of material that is lost upon filtration (determined according to the A₂₆₀ value) or tendency to form aggregates (determined according to the A₄₀₀ value), are shown in Table 2.

**TABLE 2**

| **Buffer / Solution** | **Buffer (µL) added to BO-112** | **BO-112SQ features** | | | | | |
|---|---|---|---|---|---|---|---|
| | | **Unfiltered** | | | **Post-Filtration (0.45µm)** | | |
| | | **pH** | **A₂₆₀** | **A₄₀₀** | **A₂₆₀** | **A₂₆₀ loss** | **A₄₀₀** |
| None | 0 ^{a} | 3.00 | 9.13 | 0.04 | 8.79 | 3.72% | 0.09 |
| Sodium hydroxide | 34 ^{a} | 5.00 | 9.79 | 0.34 | 9.08 | 7.25% | 0.21 |
| Citrate-phosphate | 168 ^{a} | 4.85 | 9.91 | 0.51 | 5.18 | 47.73% | 0.17 |
| Dipotassium phosphate | 30 ^{a} | 4.83 | 10.05 | 0.45 | 7.63 | 24.08% | 0.23 |
| Sodium phosphate | 230 ^{b} | 4.37 | 2.38 | 1.17 | 0.05 | - | -0.05 |
| Sodium bicarbonate (1M) | 40 ^{a} | 5.78 | 9.86 | 0.21 | 9.07 | 8.01% | 0.15 |

| | | | | | | | |
|---|---|---|---|---|---|---|---|
| ^{a} Vial containing 12 ml of BO-112 composition ^{b} Tube containing 2 ml of BO-112 composition | | | | | | | |

These experiments confirm that a potent deacidifying buffer (e.g., sodium hydroxide) is clearly able to increase the initial pH of BO-112 composition in the direction of more physiological pH values. However, an undesirable aggregation of particles was observed. To alleviate this effect, the pH of the BO-112SQ composition may be increased to values above pH 5 using sodium hydroxide with a significantly decreased loss of the Poly(I:C) molecules. However, the resulting A₄₀₀ value was 0.34, suggesting that the particle size in the BO-112SQ composition in which sodium hydroxide is added is not acceptable for clinical studies. Thus, other deacidifying buffer solutions should be tested to evaluate how a BO-112 composition for specific clinical needs can be obtained with the required features with respect to pH and particle size.

Three buffer solutions (citrate-phosphate, dipotassium phosphate, and sodium phosphate) that are commercially available at different standard concentrations (e.g., 1M, 0.5M, 0.2M, or 0.1M) and frequently used in the clinical settings, showed a less potent deacidifying effect but produced significant effects on the content of the Poly(I:C) molecules and particle aggregation, which are not compatible with an intended medical administration of the BO-112SQ compositions. However, the present data shows that sodium bicarbonate raises the pH in a BO-112SQ composition to acceptable physiological values for the intended administration of a BO-112SQ formulation, altering salient biophysical features in a more restricted manner. The exemplary BO-112SQ formulation having a volume of 12 ml contains particles in which 7.2 mg Poly(I:C) molecules are complexed with PEI. The formulation can be administered as an injectable solution in which the added volume of deacidifying solution (after a 1:300 dilution) is less than 0.4% of the BO-112 composition, and wherein buffer concentration is less than 0.03% (corresponding to less than 0.004M).

### Example 2: Biophysical and Functional Validation of BO-112SQ Formulations

### Materials & Methods

Alternative BO-112SQ formulations were generated using BO-112 compositions (Example 1) and a commercially available solution of 1.4% sodium bicarbonate for intravenous infusion (1/6M NaHCOs; Fresenius Kabi, Cat. No. 607812). Different volumes of room temperature equilibrated 1/6M NaHCO₃ (100, 200, 230, 250 and 300 µl) were added and mixed to vials (12 ml) containing the BO-112 composition as described in Example 1. Alternatively, 230 µl of 1/6M NaHCO₃ was added to a BO-112 vial using a 0.3 mL SafetyGlide Insulin syringe (0.25 mm x 8 mm, 31G; Becton Dickinson, Cat. No. 305937). Next, 2 ml aliquots of BO-112 formulation (control or deacidified with NaHCO₃) were extracted and filtered with either 0.45 µm or 0.22 µm cellulose acetate filters (Minisart syringe filters; Sartorius, Cat. No. 16555-GUK 16534-K) as described above.

A UV absorbance at 260 nm (A₂₆₀) and at 400 nm (A₄₀₀) was measured for control and test samples as described in Example 1 for comparing the structural features of the particles in the alternative BO-112SQ formulations with those of the initial BO-112 formulations. The analyses of physicochemical properties were performed immediately after adding NaHCO₃ or after incubation at room temperature for 2 hours using a Dynamic Light Scattering (DLS) equipment. Electrophoretic mobility of control BO-112 or BO-112SQ samples (unfiltered, 0.45 µm filtered, or 0.22 µm filtered) was compared with that of free Poly(I:C) molecules (10 µg of each sample plus gel loading purple dye; NEB, Cat. No. B7025S), using TAE buffer (Invitrogen; Cat. No. AM9869), pre-casted agarose gel (NZYTech; Cat. No. MB14401), SyBr-Safe dye (Invitrogen; Cat. No. S33102), and an electrophoretic system (Mini-Sub Cell GT Horizontal Electrophoresis System and PowerPac^{™} Basic Power Supply; Biorad, Cat. Nos. 1704466 and 1645050). Filtered BO-112 and BO-112SQ samples were incubated at room temperature for 24 hours. The electrophoretic mobility of the Poly(I:C) molecules was compared by loading a DNA marker (BenchTop 1kb DNA ladder; Promega, Cat. No. G7511), as a control of nucleic acid size. The concentration of the Poly(I:C) molecules in the respective BO-112 or BO-112SQ samples was later visualized and photographed using a Uvidoc Gel documentation system (UVltec; Cat. No. 286-239).

The cytotoxic activity of samples taken from the BO-112 and BO-112SQ formulations was assessed *in vitro* by a commercial assay (CellTiter 96 Aqueous NonRadioactive Cell Proliferation Assay or MTS; Promega, Cat. No. G5421), using human melanoma cell lines, in particular SK-Mel-28 (Accegen, Cat. No. ABC-TC1038) and UACC-62 (Accegen, Cat. No. ABC-TC534S) that are cultured according to provider instructions. Cells were seeded in 96 well plates (SPL, Cat. No. 30096) at a density of 3000 cells/well in a volume of 100 µl medium. Duplicate plates were prepared for each time point (24 hours and 48 hours), and quadruplicate wells were seeded for every experimental condition. Cells were placed to grow in a CO₂ incubator (Forma^{™}; ThermoFisher) at 37°C until further treatment. The unfiltered and 0.45 µm filtrated samples were incubated at room temperature for 2 hours prior to adding them to cell samples after 18 hours from cell seeding. 50 µl of cell culture medium containing 1.5 µg/ml of Poly(I:C) molecules (3x the desired final concentration i.e., 0.5 µg/ml of Poly(I:C) molecules) for each BO-112 or BO-112SQ sample were used to treat the melanoma cells. Untreated cells grown with medium only were used as a control. Following treatment, the cells were placed back to grow in the CO₂ incubator at 37°C. Cell viability was determined after a 24 hours or 48 hours treatment by adding 30 µl of MTS to the 150 µl cell culture medium. The absorbance at λ=490 nm was measured with a plate reader (X Mark Microplate Spectrophotometer; BioRad) at intervals of 15-30 minutes until the negative control (untreated cells growing with medium only) reached a value of approximately 0.8 absorbance units. The percentage of viable cells for each cell line after treatment relative to untreated cells was then calculated.

### Results

The 1M sodium bicarbonate solution that was preliminarily validated in Example 1 implies the addition of very small volumes (40 µl, 30 µl, 20 µl or less) to the standard BO-112 composition in a 12 ml vial (or its aliquots having a volume of 6 ml, 4 ml, 3 ml or less). Thus, further studies for generating alternative deacidified BO-112 formulations (BO-112SQ formulations) were performed using a 1M sodium bicarbonate solution that is diluted 1:6, or the corresponding 1/6M (or 1.4%) sodium bicarbonate injection USP solution that is commercially available for intravenous infusion. The volume of 1/6M sodium bicarbonate required to raise the pH of a 12 ml BO-112 composition to values equal to or greater than pH 5 can be more readily managed using syringes that are commonly available in the clinical setting for injecting pharmaceutical compositions having a volume of between 0.1 ml and 2.5 ml such as 0.3 ml syringes for insulin administration.

Additional experiments evaluated how optimizing and validating alternative BO-112SQ formulations that are generated using different volumes of 1/6M NaHCOs, followed (or not) by particle size exclusion using filtration through 0.2 µm and 0.45 µm filters. The effects of buffer addition on the amount of Poly(I:C) molecules and particle size were determined by UV absorbance at 260 nm (A₂₆₀) and at 400 nm (A₄₀₀) as described in Example 1, using standardized BO-112 formulations in a 12 ml volume. Sodium bicarbonate at this reduced concentration is added in a volume in a range of between less than 2% (after a 1:60 dilution in the BO-112 formulation) and 2.5% (after a 1:40 dilution in the BO-112 formulation). Accordingly, the concentration of deacidifying solution in the resulting BO-112 formulations is between less than 0.025% and 0.035% (corresponding to between less than 0.003M and 0.0042M, respectively). When the A₂₆₀ and the A₄₀₀ values are measured immediately following NaHCO₃ addition to the BO-112 formulation, both of these values increased with an increased volume of NaHCO₃ added to the BO-112 vial. Following 0.22 µm or standard 0.45 µm filtration, BO-112SQ formulations generated using up to 250 µl NaHCO₃ still present acceptable pH values of greater than 5, as shown in Table 3.

**TABLE 3**

| **Volume (µL) of 1/6M NaHCO₃ added to a 12 ml BO-112 vial** | **Features of Alternative BO-112SQ Formulations (t = 0)** | | | | | | | | |
|---|---|---|---|---|---|---|---|---|---|
| | **Unfiltered** | | | **Post-Filtration (0.22µm)** | | | **Post-Filtration (0.45µm)** | | |
| | **pH** | **A₂₆₀** | **A₄₀₀** | **A₂₆₀** | **A₂₆₀ loss** | **A₄₀₀** | **A₂₆₀** | **A₂₆₀ loss** | **A₄₀₀** |
| 0 (control) | 2.98 | 9.17 | 0.07 | 8.57 | 6.54% | 0.07 | 8.77 | 4.36% | 0.09 |
| 200 | 5.10 | 9.30 | 0.15 | 8.63 | 7.20% | 0.11 | 8.93 | 3.98% | 0.12 |
| 250 | 5.86 | 9.52 | 0.23 | 8.62 | 9.45% | 0.12 | 9.02 | 5.25% | 0.15 |
| 300 | 6.17 | 9.73 | 0.28 | 8.43 | 13.36% | 0.20 | 8.64 | 11.20% | 0.26 |

When the A₂₆₀ and A₄₀₀ values are measured after 2 hours of incubation at room temperature immediately after NaH₂CO₃ addition, the tendency for both A₂₆₀ and the A₄₀₀ values to increase with increasing volumes of NaHCOa added to the BO-112 formulation is confirmed. BO-112SQ formulations that are generated using up to 250 µl NaHCO₃ still provide acceptable pH values greater than 5 following 0.22 µm or standard 0.45 µm filtration, as shown in Table 4.

**TABLE 4**

| **Volume(µL) of 1/6M NaHCO₃ added to a 12 mL BO-112 vial** | **Features of Alternative BO-112SQ Formulations (t = 2 hours)** | | | | | | | | |
|---|---|---|---|---|---|---|---|---|---|
| | **Un filter ed** | | | **Post -Filtration (0.22µm)** | | | **Post -Filtration (0.45µm)** | | |
| | **pH** | **A₂₆₀** | **A₄₀₀** | **A₂₆₀** | **A₂₆₀ loss** | **A₄₀₀** | **A₂₆₀** | **A₂₆₀ loss** | **A₄₀₀** |
| 0 (control) | 3.00 | 9.00 | 0.09 | 8.56 | 6.65% | 0.07 | 8.79 | 4.14% | 0.05 |
| 200 | 5.48 | 9.14 | 0.13 | 8.38 | 9.89% | 0.09 | 8.72 | 6.24% | 0.07 |
| 250 | 6.47 | 9.31 | 0.20 | 8.37 | 12.08% | 0.11 | 8.91 | 6.41% | 0.09 |
| 300 | 6.86 | 9.73 | 0.35 | 8.21 | 15.62% | 0.20 | 8.91 | 8.43% | 0.24 |

The validation of appropriate NaHCO₃ volumes that allow re-formulating a BO-112 composition, such as a BO-112SQ formulation, may be pursued using the same filtration-based approach assay. An intermediate volume from 1/6M NaHCOa solution comprised between 150 µl and 250 µl (e.g., 230 µl) can be added to the standard 12 ml BO-112 formulation either using a Gilson pipette commonly used in laboratories or a 0.3 ml insulin syringe, and subsequently using a 0.45 µm filter. The addition of 230 µl of 1/6M NaHCOa increases the pH of BO-112SQ formulation to values comprised between pH 5.0 and pH 6.5, with an A₂₆₀ loss lower than 10%, and A₄₀₀ values maintained below 0.25, even after a post-filtration incubation at room temperature up to 2 hours, indicating that BO-112 particle size is not significantly altered by the addition of NaHCOa at this intermediate volume, independent from the filtration approach. If these BO-112SQ samples are tested by agarose gel electrophoresis, no significant amount of Poly(I:C) molecules is detected, confirming that the deacidification process does not trigger the release of free Poly(I:C) molecules in the formulation.

The 230 µl volume of a 1/6M NaHCO₃ solution for a 12 ml BO-112 formulation (corresponding to a NaHCOa volume of 1.9% and a NaHCOa concentration that can be expressed as 0.027% or 0.003M) was used as a reference for generating BO-112SQ formulations that were tested using more complex validation assays. The physicochemical properties of BO-112 composition and BO-112SQ samples were analyzed and compared using Dynamic Light Scattering (DLS) analysis. DLS showed an increase in the particle size of the complexes within the BO-112SQ composition upon addition of 1/6M NaHCO₃ solution immediately after deacidification and was further increased after 2 hours. However, other relevant parameters such as pH, zeta potential, hydrodynamic diameters and polydispersity characteristics were shown to meet the acceptance criteria for BO-112 formulations, even in absence of the 0.45 µm filtration, as shown in Table 5.

**TABLE 5**

| **Parameter** | **Acceptance criteria** | **(t = 0 hours)** | | **(t = 2 hours)** | |
|---|---|---|---|---|---|
| | | **BO-112** | **BO-112SQ** | **BO-112** | **BO-112SQ** |
| Hydrodynamic diameters (Z-average) | 30-250 nm | 88 nm | 154 nm | 88 nm | 192 nm |
| Polydispersity (PDI) | <0.6 | 0.19 | 0.19 | 0.20 | 0.23 |
| Zeta potential | >30mV | 40.6 mV | 39.2 mV | 40.6 mV | 30.4 mV |
| pH | 2.0-4.0 (BO-112) | 2.83 | 5.28 | 2.83 | 5.40 |
| | 5.0-6.5 (BO-112SQ) | | | | |

The biological activity of the BO-112 compositions and BO-112SQ formulations (unfiltered or after 0.45 µm filtration) were analyzed and compared using a commercial, MTS-based cytotoxicity assay and reference cancer cell lines. The percentage of viable SK-Mel-28 and UACC-62 cells (human melanoma cell lines) was tested after exposure to BO-112 and BO-112SQ formulations at 24 hours and 48 hours, using untreated cells as a negative control. The outcome of the primary *in vitro* assay for evaluating the biological activity of the BO-112 and BO-112SQ formulations confirmed in both samples that the number of viable cells was below 60% after 24 hours and below 40% after 48 hours. The number of viable cells following treatment with either the BO-112 or BO-112SQ formulation having the same Poly(I:C) concentration is highly similar regardless the pH of the formulation tested in this assay.

These results suggest that deacidification with NaHCO₃ in the BO-112SQ formulations does not qualitatively modify the core biological activity of the BO-112 composition. The assays and values described in this Example may be used as guidance for evaluating further intermediate volumes of the 1/6M NaHCO₃ solution comprised at a volume of between 150 µl and 250 µl (including 160 µl, 170 µl, 180 µl, 190 µl, 200 µl, 210 µl or 220 µl) for a 12 ml BO-112 composition, wherein these volumes correspond to intermediate values relating to the percentage and molarity of NaHCO₃ tested in this Example. Moreover, alternative buffers approved by regulatory agencies including the Food & Drug Administration (FDA) or the European Medicines Agency (EMA) and containing any suitable buffer (e.g., acetate, citrate, tartrate, histidine, glutamate, phosphate, Tris, glycine, bicarbonate, succinate, sulfate, or nitrate) may be evaluated according to the methods disclosed herein to identify suitable buffers which, at appropriate concentration ranges, yield alternative BO-112SQ formulations that present comparable features with respect to dilution, volume, molarity, A₂₆₀ and the A₄₀₀ reference values as determined when adding sodium bicarbonate to a BO-112 composition. Such alternative BO-112SQ formulations, like those based on the addition of sodium bicarbonate, may be used as exemplary BO-11XSQ formulations for a variety of clinical applications, in particular those clinical applications requiring subcutaneous, intramuscular, intratumoral, or intravascular administration of the formulation in large volumes, i.e., administered by one or more injections or infusions, and/or administered using low injection flow rates.

### Example 3: Detection and Quantification of Poly(I:C) Molecules Released in Liquid Biological Samples by Particles that are Comprised in a BO-11X Formulation

### Materials & Methods

The initial validation of the assay according to the present invention was performed using calibration stock vials of BO-111 compositions that were prepared according to the methods disclosed in WO 2017/085228. The stock solution of Poly (I:C) molecules (6.5 mg/ml) was removed from the freezer (-80°C) and thawed at room temperature. The polyethyleneimine (PEl; 150 mM) and glucose (20% w/v) stock solutions were removed from the freezer (-20°C) and thawed at room temperature. The solutions of Poly(I:C) molecules and glucose were combined to yield a final concentration of 0.414 mg/ml Poly(I:C) by mixing 26.17 µl H₂O (RNase free) 3.82 µl Poly(I:C), and 30 µl glucose. The PEI solution having a final concentration of 3.75 mM PEI was prepared by 97.5 µl H₂O

(RNase free) and 2.5 µL PEI. Equal volumes of the two solutions were mixed to prepare the BO-111 calibration reference solution in a new tube following dilution to obtain the final concentration of 0.207 mg/ml Poly I:C, 1.875 mM PEI and 5% (w/v) glucose. After rapidly mixing the two solutions, the tube was carefully examined for precipitate formation. The solution was incubated for 10 min at room temperature to allow for the formation of complexes. The aliquots were stored in a refrigerator at 3°C to 5°C until use (within 3 days). Nine calibration standards (Cal1 to Cal9, 2.66 - 304.41 ng/ml) at decreasing Poly(I:C) concentrations were prepared at room temperature by subsequently diluting the calibration standard solution with Phosphate-buffered saline (PBS; pH 7.4).

The plates and the BO-111 compositions were prepared using reagents that were tested for absence of endogenous RNase contaminating activities prior to use. A S. *aureus* protein A solution (100 µL at 10 µg/mL in 1x PBS buffer) was added to each well of a 384 - or 96-well plate and incubated over night at 5°C. The wells were washed once with 400 µL PBS buffer. The assay buffer (200 µL of 0.5% (w/v) bovine serum albumin in PBS buffer) was added to each well and the plate was incubated for 2 hours at 37°C. The wells were washed again with 400 µL PBS buffer. The first anti-dsRNA antibody (i.e., 100 µL of 1 µg/ml of IgG2a J2 antibody solution, available from e.g., Jena Bioscience) was added to each well and the plate was incubated overnight at 5°C. The wells were washed once with washing buffer (400 µL 0.5% Tween-20 in PBS buffer). An aliquot of each calibration or test sample (50 µl of the calibration standard BO-111 solutions or different preparations of BO-112 composition) was added to each well after having been mixed gently with sample diluent (i.e., heparin, sodium salt diluted at a final concentration of 0.2 mg/ml in RNase-free water) The plate was incubated for 1 hour at room temperature before washing the wells twice with 200 µL of washing buffer. The second monoclonal mouse-anti-dsRNA antibody (100 µL of 1:100 dilution of IgM K2 antibody solution, available from e.g., Jena Bioscience) was added to each well and the plate was incubated 1 hour at room temperature. The wells were washed twice with 200 µL of washing buffer prior to adding the alkaline phosphatase-conjugated anti-IgM antibody (100 µL of 1:1000 dilution of goat-anti-mouse IgM in washing buffer, available from e.g., Sigma-Aldrich and Abcam) and incubated for 1 h at 25°C. The wells were washed twice with 200 µL washing buffer and then twice again with 200 µL PBS buffer. Alkaline phosphatase yellow liquid substrate (100 µL of pNPP; available from e.g., Sigma-Aldrich) was added to each well and incubated for 20-40 min at room temperature in the dark. The reaction is stopped by adding NaOH (25 µL of 3 M NaOH) to each well and the absorbance was measured at 405 nm in a microplate reader (Tecan). A blank sample was prepared for background subtraction. Each final dilution was measured in triplicate, and, after completion of the analysis, the calibration standard dilutions were discarded. A 4-parameter mathematical fit on the calibration lines is performed by the software in the analytical instrument (Agilent BioTek Gen5 software).

An assay for the detection of the Poly(I:C) molecules was validated using a similar protocol in which the calibration samples mixed with heparin were substituted with rat or dog plasma samples taken from control animals (for blank, negative control samples) or BO-112-treated animals that were subjected to total RNA extraction using guanidine-HCl / Urea mix, proteinase K treatment, and silica-based solid phase extraction according to the Roche High Pure Viral Nucleic Acid Kit (Roche Cat. No. Cat. No. 11 858 874 001; protocol versions 16 to 20). Positive samples are prepared by spiking BO-112 composition into naive rat or dog plasma, at 5, final reference concentrations of Poly(I:C) molecules, namely, 250 ng/ml, 200 ng/ml, 150 ng/ml, 100 ng/ml, and 62.5 ng/ml; prepared in duplicates or triplicates. All plasma preparations were prepared using Aprotinin K3-EDTA tubes and stored at -80°C until use. On the day of sample preparation, plasma samples were thawed on crushed ice, mixed gently and centrifuged to collect the liquid at the bottom of the tube. A 50 µL aliquot was removed, and RNA was extracted as described in the kit leaflet. Plates and tubes may be centrifuged for washing and extracting solutions equipment using appropriate equipment (Gilson GMCLab CAPSULEFUGE PMC880 Mini Centrifuge; Hettich 50RS Rotixa Centrifuge; several centrifuge models from Agilent).

### Results

The bioanalytical methods for measuring and comparing data generated by imaging and the use of biomarkers, i.e., as established by medical and regulatory authorities for a given disease and/or patient population, are constantly evolving and improving in order to support drug development and clinical activities related to personalized medicine, in particular for treating cancer, a topic that has been extensively reviewed (Jain K, 2021; Bravo-Merodio L et al., 2021; Patel SK et al., 2020). When a BO-11XSQ formulation (or any BO-11X formulation in general) is administered to a subject, the clinical status and response to this treatment may require evaluating not only such biomarkers and imaging data, but also the actual presence of the particles comprised in this formulation within a biological sample obtained from a subject after a period of time following administration to the subject. The period of time can be defined as hours, days, or weeks post-administration. At this stage, the Poly(I:C) molecules present in a biological sample from a subject, in particular serum or plasma, may be used as a surrogate marker for the presence of the particles. However, an assay for performing this evaluation must be sufficiently reliable, reproducible, and robust in order to support the clinical research and development activities required for determining the optimal route, dosage, and/or regimen for administering the BO-11XSQ formulations (or any BO-11X formulation).

Sparse evidence in the literature underscores the opportunity for establishing and making use of anti-dsRNA antibodies directed to the double-stranded structures within Poly(I:C) molecules (Rodriguez B et al., 2011; Bokarewa M et al., 2008; Bonin M et al., 2000; Schonborn J et al., 1991), but without establishing any specific assay for quantifying Poly(I:C) molecules in a biological sample after clinical administration, let alone when Poly(I:C) molecules are administered in complex forming particles as for BO-11XSQ formulation (and BO-11X formulations in general). This modality of administration requires not only identifying the appropriate conditions for disaggregating the particles present in the biological samples, but also for determining low or trace amounts of Poly(I:C) molecules (e.g., below 100 ng/ml, possibly below 50 ng/ml) such that the timeframe during which the particles containing Poly(I:C) molecules are present and/or if administering higher doses of a BO-11XSQ or BO-11X formulation actually increase the presence of such particles in tissues, blood, or specific fractions (such as plasma) can be determined.

A preliminary study for identifying and quantifying the Poly(I:C) molecules was performed using a lab-scale, manual preparation of BO-11X (BO-111; WO 2017/085228) to establish the feasibility of an assay having the ELISA format for detecting and quantifying Poly(I:C) molecules in the original pharmaceutical composition. A controlled disaggregation of the particles in this composition is achieved by diluting an aliquot of a BO-111 composition using a solution containing heparin (0.2 mg/ml in RNase-free water). The liquid sample is then incubated in commercially available plates for performing an ELISA assay that presents similar sizes and generally contain 24, 48, 96, or more wells, thereby having wells that allow for using a wide range of liquid volume. The plates are previously prepared by subsequently immobilizing Protein A from *Staphylococcus aureus,* blocking the surface of the wells for unspecific binding using albumin, and then coating them with a commercial monoclonal anti-dsRNA antibody (J2; IgG type) that is bound by Protein A. After incubating and washing away the heparin-diluted BO-111 aliquot, the wells covered by the Poly(I:C) molecules are incubated with a further commercial monoclonal mouse anti-dsRNA antibody (K2; IgM type). The latter antibody is then detected using a goat antimouse IgM alkaline phosphatase conjugate. A commercial kit allows detecting and quantifying this enzymatic activity, which produces an absorbance signal at 405 nm that can be detected using a multiplate reader.

The data generated using different concentrations of BO-111 (and thus of Poly(I:C) molecules) are then used to establish a calibration profile using analytical software and instruments within a relevant range of Poly(I:C) concentrations. This approach allows for establishing a calibration curve showing a strict linearity between 2.66 ng/ml (LLOQ; signal-to-noise 4:1) and 10.14 ng/mL (ULOQ). This ELISA assay is then tested using aliquots of GMP-manufactured BO-112 compositions, thereby confirming the reliability of the instant approach for quantifying dsRNA in Poly(I:C) molecules as a surrogate measure of Poly(I:C)-containing particles in a liquid sample.

The present ELISA-based method has been tested and optimized using plasma obtained from animals (dogs or rats) from which blood samples were obtained before and after administering a BO-112 composition. Distinct study groups of animals were established where the BO-112 composition is administered intravenously to the animals at different dosages (e.g., between 0.09 mg/kg to 1.0mg/kg in Wistar rats; between 0.1 mg/kg to 0.4 mg/kg in Beagle dogs) between 4 and 8 times during a period of 4 weeks (e.g., weekly at day 1, 8, 15, and 22, or bi-weekly at day 1, 4, 8, 11, 15, 18, 22 and 25). The blood samples are taken at different time points up to 4 hours following administration of the formulation (e.g., at 5 min, 15 min, 30 min, 1 hour, 2 hours, 3 hours, and 4 hours).

The *in vitro* assay described herein for the BO-111 and BO-112 formulations was performed using, instead of heparin, a commercial kit used for RNA extraction. The kit contains materials and instructions for preparing RNA from biological samples (e.g., plasma) using a solution that contains guanidinium chloride and urea. The RNA preparations obtained using this kit were then tested in the ELISA assay described above, which showed a significant increase in Poly(I:C) molecules, and thus of circulating particles after BO-112 administration that is proportional to the dose of BO-112 composition and inversely proportional to the period of time from the administration. For instance, in the aforementioned study performed in dogs, the assay revealed a peak in the concentration of Poly(I:C) molecules in the plasma of between 5 min and 30 min after BO-112 administration for all dosage groups ranging from 65.95 to 443.71 ng/ml as the highest detectable amount, while the lowest detectable amounts, i.e., at lower doses and/or at least 1 hour after administration, were about 20-25 ng/ml of the Poly(I:C) molecules.

Collectively, the combined use of the disclosed RNA detection kit and of the ELISA platform initially established using samples of the BO-111 and BO-112 compositions advantageously allows for determining the presence of the particles comprised in the BO-11XSQ and BO-11X formulations subsequent to administration, in particular within plasma, when administered intravenously. The presently disclosed assay therefore beneficially allows for a highly efficient and precise evaluation of the biomedical effects of BO-11X formulations at different dosages and regimens.

The present invention provides a number of improved metrics for validating the dosages and administration regimens for BO-11X compositions, and in particular BO-11XSQ formulations, which present a variety of improved characteristics making it possible to safely administer these formulations in a human subject with the most effective administration frequency and/or at high dosages, in particular when the compositions are injected subcutaneously, intramuscularly, or intravenously. This approach may be also used to evaluate how Poly(I:C) preparations useful for preparing BO-11X compositions and BO-11XSQ formulations, such as those described in WO 2017/085228 or WO 2018/210439 on the basis of the size of the disclosed Poly(I) and Poly(C) components and the ranges of the resulting Poly(I:C) molecules. The concentration of the Poly(I:C) molecules, the volume of the injected formulation, and the buffers or other components (e.g., diluents, excipients, sugars, or stabilizing agents) may be appropriately tested and combined in order to obtain Poly(I:C)-only compositions having the desire pharmaceutical or medical features.

### REFERENCES

Agrawal E and Kandimalla R, 2019. Immuno-Oncology Tech. 3: 15-23.
Aznar M et al., 2019. Immunother Cancer.7: 116.
Badkar A et al., 2021. Drug Des Dev Ther. 15: 159-170.
Bishani A and Chernolovskaya E, 2021. Int J Mol Sci. 22: 13360.
Bonin M et al., 2000. RNA. 6: 563-570.
Bokarewa M et al., 2008. Eur J Immunol. 38: 3237-3244.
Bravo-Merodio L et al., 2021. Adv Clin Chem. 102:191-232.
Fu X et al., 2023. Genes Dis. 11: 306-320.
Gutierrez L et al., 2020. Jour Pharm Sci. 109: 3524-3534.
Huang Let al., 2022. Pharmaceutics 14, 1228.
Jain K, 2021. Textbook of Personalized Medicine (3rd Edit.) Springer Nature Switzerland AG.
Kaza M et al., 2019. J Pharm Biomed Anal. 165: 381-385.
Lang D et al., 2020. J Clin Med. 9: 3483.
Lee K et al., 2023. Cells. 12: 2147.
Liberini V et al., 2021. Molecules. 226:2201.
Márquez-Rodas I et al., 2022. Cancer Res. 82 (12_Supplement): CT014.
Patel SK et al., 2020. Front Pharmacol. 11: 1177.
Rodriguez B et al., 2011. BMC Cancer. 11: 110.
Sankar P et al., 2019. Int. J. Pharm. Sci. Rev. Res. 56: 50-58.
Schonborn J et al., 1991. Nucleic Acids Res. 19: 2993-3000.
Usach I et al. 2019. Adv Ther. 36, 2986-2996.
van de Donk P et al., 2020. Theranostics. 10: 1708-1718.
Xiong H et al., 2021. Int J Mol Sci. 22: 3295.
Zahavi D and Weiner L, 2020. Antibodies (Basel) 9: 34.

## Claims

1. An aqueous formulation, comprising particles formed by a combination of polyinosinic-polycytidylic acid [Poly(I:C)] molecules and polyethyleneimine, wherein the formulation comprises a pH of between pH 5.0 and pH 7.5.

2. The aqueous formulation according to claim 1, wherein the particles comprise a pH of between pH 2.0 and pH 4.0, and wherein the particles further comprise a deacidifying solution comprising a pH of between pH 7.0 and pH 10.0.

3. The aqueous formulation according to claim 2, wherein the volume of the deacidifying solution is between 0.01 % and 5.0 % of the formulation comprising the particles.

4. The aqueous formulation according to claim 2 or 3, wherein the molarity of the deacidifying solution in the formulation is less than 0.01M.

5. The aqueous formulation according to any of the preceding claims, wherein the deacidifying solution comprises sodium bicarbonate.

6. The aqueous formulation according to any of the preceding claims, wherein the concentration of Poly(I:C) molecules is at least 0.5 mg/ml and/or the amount of Poly(I:C) molecules is between 0.1 mg and 25 mg.

7. The aqueous formulation according to any of the preceding claims, further comprising excipients, sugars, stabilizing agents, labelling agents, organic solvents, preservatives, adjuvants, and/or drugs.

8. A kit for preparing an aqueous composition according to any of claims 1-7 comprising an aqueous formulation formed by a combination of Poly(I:C) molecules and polyethyleneimine, and a deacidifying solution, wherein the deacidifying solution preferably comprises sodium bicarbonate.

9. An aqueous formulation comprising particles comprising Poly(I:C) molecules and polyethyleneimine according to any of claims 1-7 for use as a medicament.

10. An aqueous formulation comprising particles comprising Poly(I:C) molecules and polyethyleneimine according to any of claims 1-7 for use in therapy, wherein the therapy is stimulating an immune response, treating or preventing cancer, or a vaccine therapy.

11. The aqueous formulation for use according to claim 9 or 10, wherein the formulation is administered in combination with a second pharmaceutical composition.

12. The aqueous formulation for use according to claim 11, wherein the second pharmaceutical composition is selected from a vaccine, an anti-cancer treatment, and a standard-of-care medical treatment.

13. The aqueous formulation for use according to claim 12, wherein the anti-cancer treatment is selected from surgery, radiotherapy, tumor ablation, cryotherapy, laser therapy, and phototherapy.

14. The aqueous formulation for use according to any of claims 11 to 13,
wherein the second pharmaceutical composition comprises an anti-cancer agent, wherein the anti-cancer agent is preferably selected from a chemotherapeutic agent, a cancer vaccine, an immune-modulating agent, and a cell-based product, wherein the immune-modulating agent is preferably an anti-PD1 antibody or an anti-PD-L1 antibody.

15. The aqueous formulation for use according to any of claims 10 to 14,
wherein the formulation and/or second pharmaceutical composition is administered by oral, subcutaneous, intradermal, intratumoral, intravenous, intramuscular, intrathecal, intranasal, intravesical, intra-articular, topical, or transdermal administration, and
wherein the formulation and the second pharmaceutical composition is preferably administered simultaneously or sequentially to the subject.
